# EUROPEAN PATENT SPECIFICATION

(11) **EP 1 105 359 B1**
(45) Date of publication and mention of the grant of the patent: **30.10.2002**
(21) Application number: 99943811.2
(22) Date of filing: 19.08.1999
(51) Int. Cl.: C07B 37/04, C07C 235/84

(54) **SOLID PHASE SYNTHESIS OF COMPOUNDS BY CARBON-CARBON BOND FORMING MITSUNOBU REACTIONS**
FESTPHASESYNTHESE VON VERBINDUNGEN MITTELS MITSUNOBU-REAKTIONEN UNTER BILDUNG VON KOHLENSTOFF KOHLENSTOFF BINDUNGEN
SYNTHESE EN PHASE SOLIDE DE COMPOSES PAR REACTIONS MITSUNOBU DE FORMATION DE LIAISON CARBONE-CARBONE

(30) Priority: 21.08.1998 US 138433
(43) Date of publication of application: 13.06.2001
(73) Proprietor: PE Corporation (NY), Foster City California 94404 (US)
(72) Inventor: CHATURVEDI, Surendrakumar, San Ramon, CA 94583 (US)
(74) Representative: Vossius, Volker, Dr.
(86) International application number: US9919076
(87) International publication number: WO00010942

(56) References cited:
- EP-A- 0 291 199
- WO-A-96/40201
- T. A. RANO: "Solid phase synthesis of aryl ethers via the Mitsunobu reaction" TETRAHEDRON LETTERS, vol. 36, no. 22, 1995, pages 3789-3792, XP004027982 OXFORD GB cited in the application
- M. WADA: "Intermolecular dehydration between alcohols and active hydrogen compounds by means of diethyl azodicarboxylate and triphenylphosphine" TETRAHEDRON LETTERS, no. 13, 1972, pages 1279-1282, XP002123592 OXFORD GB cited in the application
- O. MITSUNOBU: "The use of diethyl azodicarboxylate and triphenylphosphine in synthesis and transformation of natural products" SYNTHESIS,1981, pages 1-28, XP002123593 STUTTGART DE cited in the application

## Description

### FIELD OF THE INVENTION

The invention relates generally to the field of organic chemistry using combinatorial synthesis methods. More specifically, the invention relates to conducting Mitsunobu carbon-carbon bond forming reactions on solid-phase beads and surfaces.

### REFERENCES

Baldwin, J., Ohlmeyer, and Henderson, I. "Combinatorial sulfonamide library", US Patent 5,618,825, issued April 8, 1997.
Baldwin, J., Henderson, I. and Waksmunski, F. "Combination hydroxypropylamine library", US Patent 5,766,963, issued June 16, 1998.
Brenner, S. and Lerner, R. "Encoded combinatorial chemistry", Proc. Natl. Acad. Sci. USA 89:5381-83 (1992).
Caruthers, M. and Matteucci, M. "Process for preparing polynucleotides", US Patent No. 4,458,066, issued July 3, 1984.
Dankwardt, S., Smith, D., Porco, J. and Nguyen, C. "Solid Phase Synthesis of N-Alkyl Sulfonamides", Synlett. 854-56 (1997).
Desai, M., Nuss, J., Spear, K., Renhowe, P., Brown, E., Richter, L. and Scott, B. "Combinatorial libraries of substrate-bound cyclic organic compounds", WO 96/40201, Intl. Publ. Date: 19 December 1996.
Dower, W., Barrett, R. and Gallo, M. and Needels, M. "Synthesizing and screening molecular diversity", US Patent 5,639,603, issued June 17, 1997.
Ellman, J. "Solid phase and combinatorial synthesis of benzodiazepine compounds on a solid support", US Patent 5,288,514, issued Feb. 22, 1994.
Ellman, J. "Solid phase and combinatorial synthesis of compounds on a solid support", US Patent 5,545,568, issued Aug. 13, 1996.
Fields, G. and Noble, R. "Solid phase peptide synthesis utilizing 9-fluorenylmethoxycarbonyl amino acids", Int. J. Peptide Protein Res. 35:161-214 (1990).
Frechet, J. "Synthesis and applications of organic polymers as supports and protecting groups", Tetrahedron 37:663-83 (1981).
Furka, A., Sebestyen, F., Asgedom, M., and Dibo, G. "General method for rapid synthesis of multicomponent peptide mixtures", Int. J. Peptide Protein Res. 37:487 (1991).
Gallop, M and Murphy, M. "Methods for synthesizing diverse collections of pyrrolidine compounds", US Patent 5,525,735, issued June 11, 1996.
Geiser, T., Beilan, H., Bergot, J. and Otteson, K. "Automation of solid-phase peptide synthesis" in *Macromolecular Sequencing and Synthesis,* Alan R. Liss, Inc., 1988, pp. 199-218.
Greene, T. and Wuts, P. in *Protective groups in Organic Synthesis*, 2^{nd} ed., Wiley, 1991. Hodge, P., "Polymer-supported organic reactions: what takes place in the beads?", Chem. Soc. Reviews 26:417-24 (1997).
Houghten, R. *et al.*, "Generation and use of synthetic peptide combinatorial libraries for basic research and drug discovery", Nature 354:84-86 (1991).
Hughes, D., "The Mitsunobu Reaction" in *Organic Reactions*, Vol. 42, Beak, P. *etal,* Eds., John Wiley & Sons, Inc., New York, pp. 335-656.
Ito, S. and Tsunoda, T., "Application of the aza-Claisen rearrangement to the total synthesis of natural products: (-)-Isoiridomyrmecin", Pure & Appl. Chem. 66:2071-74 (1994).
Kaiser, E. "Color test for detection of free terminal amino groups in the solid-phase synthesis of peptides", Anal Biochem. 34:595 (1970).
Katsuyama, I., Ogawa, S., Yamaguchi, Y., Funabiki, K., Matsui, M., Muramatsu, H. and Shibata, K., "A convenient and regioselective synthesis of 4-trifluoromethylpyridines", Synthesis 1321-24 (1997).
Macor, J. and Wehner, J., "The use of *O*-nitroarylacetonitriles as carbon acid participants in the Mitsunobu reaction", Tet. Letters 32:7195-98 (1991).
Marzinzik, A. and Felder, E., "Solid support synthesis of highly functionalized pyrazoles and isoxazoles; scaffolds for molecular diversity", Tet. Letters 37:1003-06 (1996)
Mitsunobu, O., "The use of diethyl azodicarboxylate and triphenylphosphine in synthesis and transformation of natural products", Synthesis 1-27 (1981).
Pauling, L., "The Chemical Bond", Cornell University Press, Ithaca, New York, 1967, pp. 63-65.
Rano, T. and Chapman, K., "Solid phase synthesis of aryl ethers via the Mitsunobu reaction", Tet. Letters 36:3789-92 (1995).
Rink, H., "Solid synthesis of protected peptide fragments using a trialkoxydiphenylmethylester resin", Tet. Letters 28:3787-90 (1987).
Saxena, A., Saxena, M., "Developments in anticonvulsants", Progress in Drug Research 44:185-291 (1995).
Tsunoda, T., Otsuka, J., Yamamiya, Y., and Ito, S., "*N,N,N',N'*-tetramethylazodicarboxamide (TMAD), a new versatile reagent for mitsunobu reaction. Its application to synthesis of secondary amines", Chem. Letters 539-42 (1994).
Tsunoda, T., Yamamiya, Y., Kawamura, Y. and Ito, S., "Mitsunobu acylation of sterically congested secondary alcohols by *N,N,N',N'*-tetramethylazodicarboxamidetributylphosphine reagents", Tet. Letters 36:2529-30 (1995).
Tsunoda, T., Nagaku, M., Nagino, C., Kawamura, Y., Ozaki, F., Hioki, H., and Ito, S., "Carbon-carbon bond formatin with new Mitsunobu reagents", Tet. Letters 36:2531-34 (1995).
Wada, M. and Mitsunobu, O., "Intermolecular dehydration between alcohols and active hydrogen compounds by means of diethyl azodicarboxylate and triphenylphosphine", Tet. Letters 1279-82 (1972).
Watson, S., Wilson, R., Judd, D., and Richards, S., "Solid phase synthesis of 5-aminopyrazoles and derivatives", Tet. Letters 38:9065-68 (1997).
Zuckermann, R., Kerr, J., Kent, S., Moos, W., "Efficient method for the preparation of peptoids [oligo(N-substituted glycines)] by submonomer solid synthesis", J. Amer. Chem. Soc. 114:10646-47 (1992).

### BACKGROUND

Many synthetic organic reactions have been applied to solid particles, beads, and surfaces (Frechet, 1981; Ellman, 1994; Ellman, 1996). The benefits of conducting multi-step syntheses are well appreciated and the methods well-defined and optimized for biopolymers, such as peptides (Geiser, 1988) and oligonucleotides (Caruthers, 1984). In fact, for these essential molecular biology tools, automated, solid-phase synthesis is the only viable approach. Like nucleic acids and peptides, other biopolymer mimics and analogs have been synthesized by automation on solid supports by repeated addition of monomer units (Zuckerman, 1992). Conducting chemical reactions on solid supports has several important and practical advantages: (i) excess reagents and soluble by-products can be easily removed and separated by simple washing and filtration steps, (ii) dispensing, manipulating, organizing the parallel production of many compounds is facilitated, and (iii) reactions can be scaled-down for economy and ease of handling.

Of all the chemical reactions available to synthesize compounds, those that make carbon-carbon bonds are the most useful. Whereas hundreds of synthetic methods are available to interconvert functional groups, e.g. esterify a carboxylic acid to a carboxylate ester, with reproducible and predictable ease, fewer methods are available to form carbon to carbon bonds. Desirable properties of carbon-carbon bond forming reactions include: (1) high yield, (2) stereoselectivity, (3) enantioselectivity, (4) safe and controlled conditions, (5) inexpensive reagents, and (6) scale-up efficiency.

A particularly powerful carbon-carbon bond forming reaction is the "Mitsunobu" alkylation reaction. The classic Mitsunobu reaction (Mitsunobu, 1981) employs a trivalent phosphorus reagent **1** and an azo-containing reagent **2** to activate a functional group (e.g. hydroxyl) of reactant **3.** Reaction with a nucleophile reactant **4** (e.g. RCO₂H, RCOSH, R₂NH, ArOH, TsOH, R₃SiOH, halide) then forms the Mitsunobu product **5** (Scheme 1).

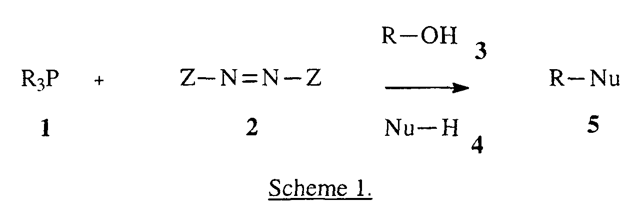

Mitsunobu reactions are usually conducted with acidic nucleophiles such as carboxylic acids (Nu = RCO₂⁻). An obvious advantage of Mitsunobu reactions over other carbon-carbon bond forming methods is that no prior activation of either reactant is necessary. The simple combination of the azo-containing reagent and the trivalent phosphorus reagent suffice, and in the case of esters, proceeds rapidly and in high yield.

However, the Mitsunobu alkylation reaction when conducted by conventional solution-phase methods is cumbersome and inefficient (Wada, 1972). The desired product is usually contaminated by a number of soluble side products including the bis-alkylation, O-alkylation, and azo-alkylated products (Mitsunobu, 1981). In fact, the bis-alkylation side product is often the major product of the reaction.

Thus, the technical problem underlying the present invention is to provide a process for forming carbon-carbon bonds on solid supports by the Mitsunobu alkylation reaction. Desirable features include (i) efficient loading of supports with appropriate functionality, (ii) efficient and high yield Mitsunobu alkylation reaction, (iii) efficient cleavage of products from the support, (iv) parallel, automated synthesis of loading, reactions, and cleavage, and (v) screening of libraries in either solution phase or on supports.

### SUMMARY

The present invention is directed toward a novel process and reagents for conducting a Mitsunobu alkylation reaction on a solid phase support. The support-bound Mitsunobu alkylation products are well suited for use as intermediates for high-throughput synthesis and screening of biologically active compounds.

In a first aspect of the invention, a solid phase support is loaded with compounds to prepare a support-bound hydroxyl reagent and a support-bound active methylene reagent. According to this aspect of the invention, a functionalized solid phase support is reacted or derivatized with reagents to provide hydroxyl and active methylene functional groups.

A first embodiment of this aspect of the present invention comprises a process for forming a carbon-carbon bond between a solid phase support-bound hydroxyl reagent and a soluble active methylene reagent (Scheme 3). A second embodiment of this aspect of the present invention comprises a process for forming a carbon-carbon bond between a solid phase support-bound active methylene reagent and a soluble hydroxyl reagent (Scheme 4). Each process is conducted with a trivalent phosphorus reagent, and an azo-containing reagent and forms a Mitsunobu alkylation product.

In a second aspect of the invention, impurities and excess reagents are separated and removed from solid support bound products by filtering, aspirating, withdrawing liquids, precipitating, or evaporating the products or the impurities and excess reagents.

In a third aspect of the invention, the support-bound products of a solid phase Mitsunobu alkylation are cleaved from the solid support with acidic, basic, or other cleavage reagents, such as fluoride. The resulting cleaved products possess a broad variety of molecular diversity and utility.

In a fourth aspect of the invention, a broad variety of Mitsunobu product compounds are synthesized in parallel using manual methods or robotic synthesis technology. The present invention is useful for the generation and rapid screening of a large number of chemically diverse new compounds. Mitsunobu alkylation products are useful intermediates for biologically active and industrially important compounds. Small molecules synthesized by the methods of the present invention may be used to form arrays which may be screened for binding affinity as ligands in cell- and protein-receptor assays.

In a fifth aspect, the present invention includes support-bound, Mitsunobu alkylation products having the structures **I, II,** and **III** 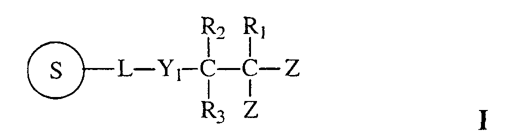 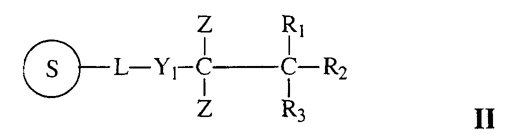 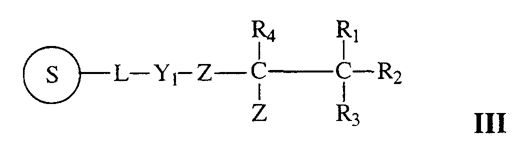 wherein S is a solid support, L is a linker, Y₁ is substituted lower alkyl and substituted aryl, R₁, R₂, R₃, and R₄ are H, lower alkyl, aryl, and halogen, and Z is an electron-withdrawing group.

These and other features and advantages of the present invention will become better understood with reference to the following definitions, figures, description, examples, and appended claims.

### DEFINITIONS

Unless stated otherwise, the following terms and phrases as used herein are intended to have the following meanings:
The term "lower alkyl" refers to straight-chain, branched, or cyclic groups consisting of 1-20 carbon atoms. "Alkenyl" refers to straight-chain, branched, or cyclic groups consisting of 2-20 carbon atoms and at least one double bond. "Alkynyl" refers to straight-chain, branched, or cyclic groups consisting of 2-20 carbon atoms and at least one triple bond. "Aryl" refers to phenyl, thienyl, furanyl, pyridyl, pyrimidyl, naphthyl, fluorenyl, biphenyl, phenoxyphenyl, benzyloxyphenyl, trityl, fluorenyl, anthracenyl, xanthenyl, and substituted forms thereof.
The term "substituted" refers to replacement of up to three H atoms on a carbon by a non-hydrogen atom or moiety, e.g. halogen, hydroxy, lower alkoxy, carboxy, carboalkoxy, carboxamido, cyano, carbonyl, nitro, amino, alkylamine, alkylthio, sulfoxide, sulfone, acylamino, amidino, phenyl, benzyl, heteroaryl, phenoxy, benzyloxy, and heteroaryloxy.
The terms "library", "combinatorial" and "combinatorial library" all refer to a collection of single compounds, or mixtures of compounds, synthesized on a solid support from a plurality of reagents by reactions conducted in a largely parallel format (Gallop, 1996).

The phrase "split and mix" refers to a combinatorial synthesis method whereby solid supports, typically beads, are reacted with reagents to contain successive precursors to the target compounds that form the library and alternately combined, separated, and distributed (Furka, 1991).

"Linker" refers to one or more atoms comprising a chain connecting a solid support to a chemically reactive moiety.

The terms "electron-withdrawing" and "electron-withdrawing group" refer to an electronegative functional group which draws electrons away from a reactive site of a molecule. "Electronegativity" is defined according to the Pauling Index (Pauling, 1967). Electron-withdrawing groups as used here are electronegative relative to a methylene carbon. For example, diethyl malonate has electron-withdrawing ester groups which increase the acidity, and thus the nucleophilicity, of the methylene carbon. In this example the "active methylene" of diethyl malonate is activated by the electron-withdrawing ester groups.

### BRIEF DESCRIPTION OF THE FIGURES

- Figure: 1 shows the coupling of an amino functionalized support, e.g. polystyrene-Rink linker, with carboxylic acid compounds **18-23** to form hydroxyl containing supports **24-29.**
- Figure 2: shows Mitsunobu alkylation of an hydroxyl-containing support **24** to form support compounds **30-36.**
- Figure 3: shows compounds **37-43** cleaved from supports **30-36.**
- Figure 4: shows the synthesis of 11-hydroxy-4-(methoxy(hydroxyphosphoryl))-2,4-dimethylundecan-3-one by coupling 2-chlorotritylchloride-polystyrene and 1,6-hexanediol followed by Mitsunobu alkylation with 4-dimethoxyphosphoryl-2-methylpentan-3-one. The product is mono-demethylated under the acidic cleavage conditions.

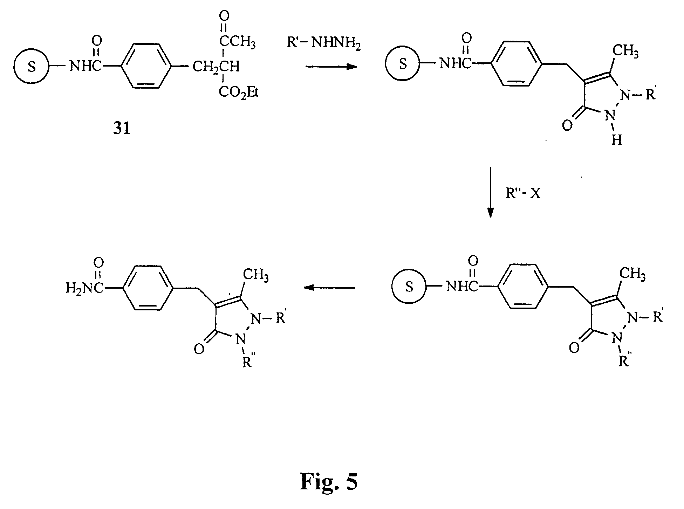

- Figure 5: shows the synthesis of pyrrazolinone compounds from Mitsunobu alkylation product **31** which result from cyclization with hydrazine compounds and further N-alkylation.
- Figure 6: shows the synthesis of pyrimidine derivatives, useful as antiviral agents, and 1-aminopyrrazolinone compounds, from Mitsunobu alkylation product **32.**
- Figure 7: shows the synthesis of phenobarbital compounds, useful as psychoactive agents, from Mitsunobu alkylation product **30**, after alkylation.
- Figure 8: shows the synthesis of a 16 reaction vessel array of anticonvulsant compounds entailing the steps of loading the polystyrene-Rink amide support, Fmoc cleavage, coupling of carboxylic acid-hydroxyl reagents, Mitsunobu alkylation, and cleavage of 16 unique compounds from the support in a spatially addressable format.

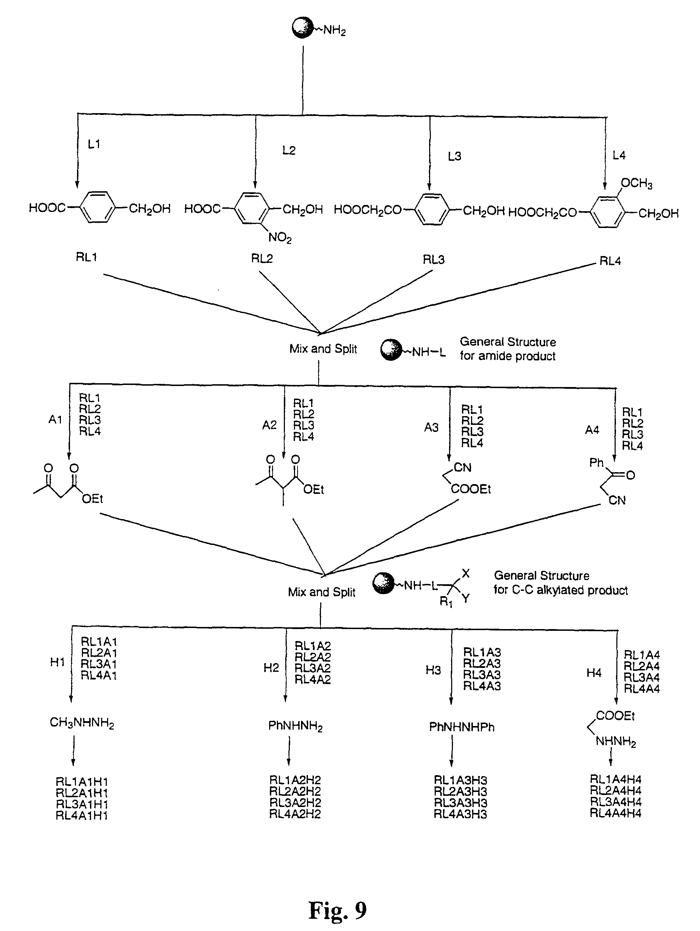

- Figure 9: shows the synthesis of 64 unique compounds by a 16 reaction flask array. The polystyrene-Rink linker support is placed in 16 vessels. After each reaction, the support is combined, mixed, and divided back into the vessels. In this manner, greater structural diversity is attained by synthesizing mixtures in each reaction flask than parallel synthesis of one compound per reaction flask. At the end of the synthetic process of loading the support with carboxylic acid-hydroxyl reagents, Mitsunobu alkylation, cyclization with hydrazine reagents, a mixture of 16 compounds are in each of the 16 flasks.

### DETAILED DESCRIPTION OF THE PREFERRED EMBODIMENTS

Reference will now be made in detail to the preferred embodiments of the invention, examples of which are illustrated in the accompanying figures and schemes. While the invention will be described in conjunction with the preferred embodiments, it will be understood that they are not intended to limit the invention to those embodiments. On the contrary, the invention is intended to cover all alternatives, modifications, and equivalents, which may be included within the invention as defined by the appended claims.

Generally, the present invention comprises processes for Mitsunobu alkylation on solid supports. Additionally, the present invention comprises Mitsunobu alkylation products on solid-supports. The processes are generally conducted by bringing liquid reagents into contact with a solid support for a period of time for the Mitsunobu alkylation reaction to proceed to completion, followed by removal of excess reagents and isolation of the solid support.

A Mitsunobu solid-phase alkylation reaction forms a carbon-carbon bound between a support with hydroxyl or active methylene functionality and a soluble reagent with hydroxyl or active methylene functionality. The solid-support, 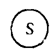 , or "support" is a reactant in the process and can be a substrate for reactions prior and subsequent to the Mitsunobu reaction step. The support is comprised of materials having a rigid or semi-rigid character and may be any shape, e.g. spherical, as in beads, rectangular, irregular particles, resins, gels, microspheres, or substantially flat. In some embodiments, it may be desirable to physically separate synthesis regions on the support with, for example, wells, raised regions, dimples, pins, trenches, rods, pins, inner or outer walls of cylinders, and the like. The spatial arrangement of the synthesis regions is termed an "array", e.g. a two-dimensional surface addressable by a programmed, robotic automated liquid delivery apparatus. As used in the methods of the present invention, supports are attached by covalent bonds, ionic bonds, or other affinity interactions, to chemically reactive compounds.

Preferred support materials include polystyrene, controlled-pore-glass, polyacrylate, hydroxethylmethacrylate, polyamide, polyethylene, polyethyleneoxy, or copolymers and grafts of such. A particularly preferred solid support of the present invention, and used extensively for small molecule and peptide synthesis (Fields, 1990), is polystyrene in bead form, cross-linked with 1-2% divinylbenzene, such as the Merrifield chloromethyl polystyrene (Scheme 2). Hydrophobic organic solvents swell this support to many times its dry volume. This gel-like state is essential for reactive species in solution to gain access to the reactive sites in the beads (Fields, 1990). The extent of swelling decreases as the percentage of crosslinking increases (Hodge, 1997).

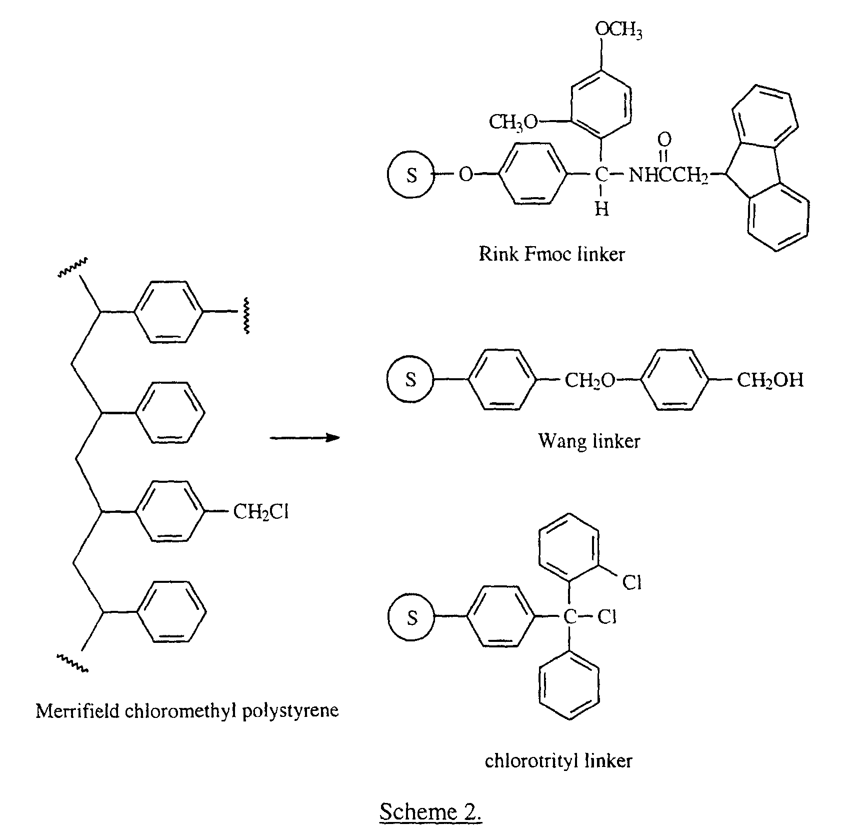

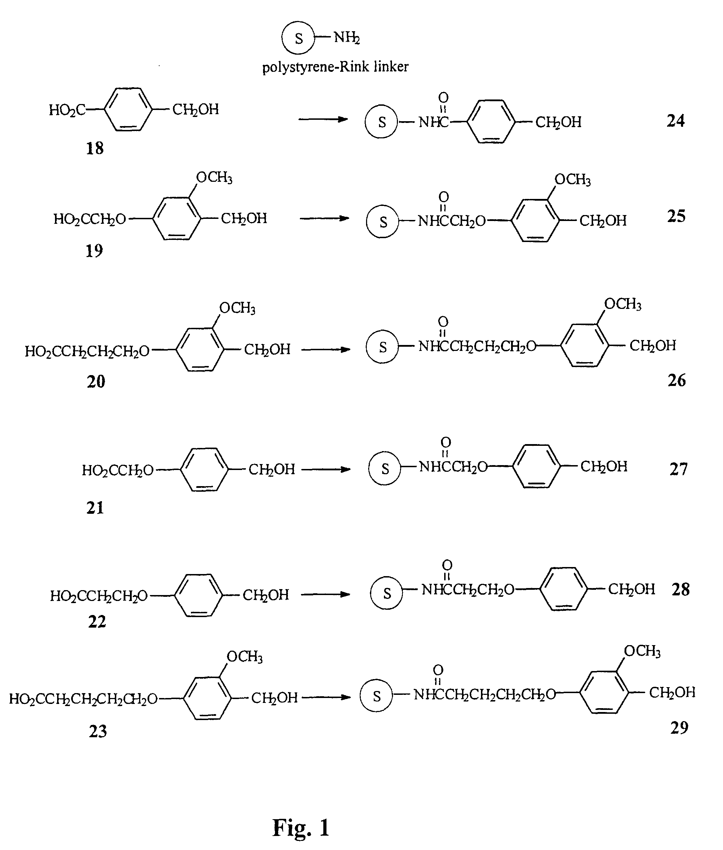

Functionalized solid supports **7** are created by reacting the solid support **6** with reagents to provide functionality such as protected amines, e.g. Rink Fmoc linker, hydroxyl, e.g. Wang linker, or chloro, e.g. chlorotrityl linker (Scheme 2). The linker, L, connects the support to the chemically reactive moiety. L can be amino, oxygen, sulfur, or a functional group such as ester, amide, sulfonate, and the like. The substituent Y₁ in **7** connects the linker, L, of the support to the functionality that reacts in the Mitsunobu alkylation reaction (Scheme 3). Y₁ is a group such as substituted lower alkyl, substituted alkenyl, substituted alkynyl, and substituted aryl. Also in **7,** R₂ and R₃ are H, lower alkyl, aryl, and halogen substituents. Specific examples of general support **7** include supports **24-29** (Figure 1). In preparation of functionalized supports for the Mitsunobu alkylation, the polystyrene-Rink Fmoc linker (Scheme 2) is treated with 20% piperidine in dimethylformamide to remove the Fmoc group and provide the amino functionality (L). Alternatively, the Wang linker (Scheme 2) bears a hydroxyl group for conversion to an ester upon coupling with a carboxylic acid. Coupling of the support-amino group of the Rink linker with the carboxylic acid activated by a carbodiimide reagent, e.g. diisopropylcarbodiimide under standard amide forming conditions, with a variety of carboxylic acid-hydroxyl compounds (e.g. **18-23**) provides the hydroxyl functionality supports **24-29** (Figure 1 and Examples 1-7). The reactions can be monitored for disappearance of free amine by the Kaiser or ninhydrin colormetric tests. Gentle agitation of the support by mechanical shaking facilitates optimal loading.

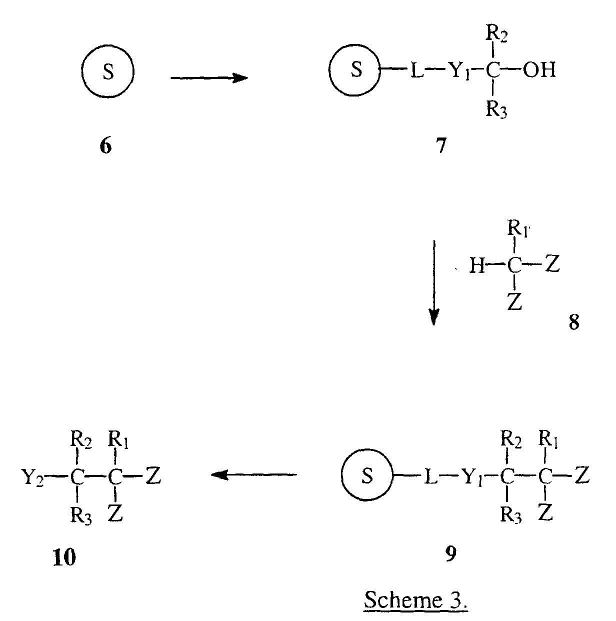

Similarly, supports **11** and **12** may be prepared from **6** where R₄ is H, lower alkyl, aryl, and halogen substituents (Scheme 4). Supports **11** and **12** bear active methylene groups for reaction with soluble hydroxyl reagents **13.** Supports **11** and **12** differ by the electron-withdrawing group Z in a non-bridging (**11**) or bridging (**12**) arrangement.

The loading range for functionality on **7, 11,** and **12** is typically 0.05 to 1.5 mmol per gram of support. The preferred loading range is 0.2 to 0.7 mmol per gram, and the most preferred loading range is 0.25 to 0.40 mmol per gram.

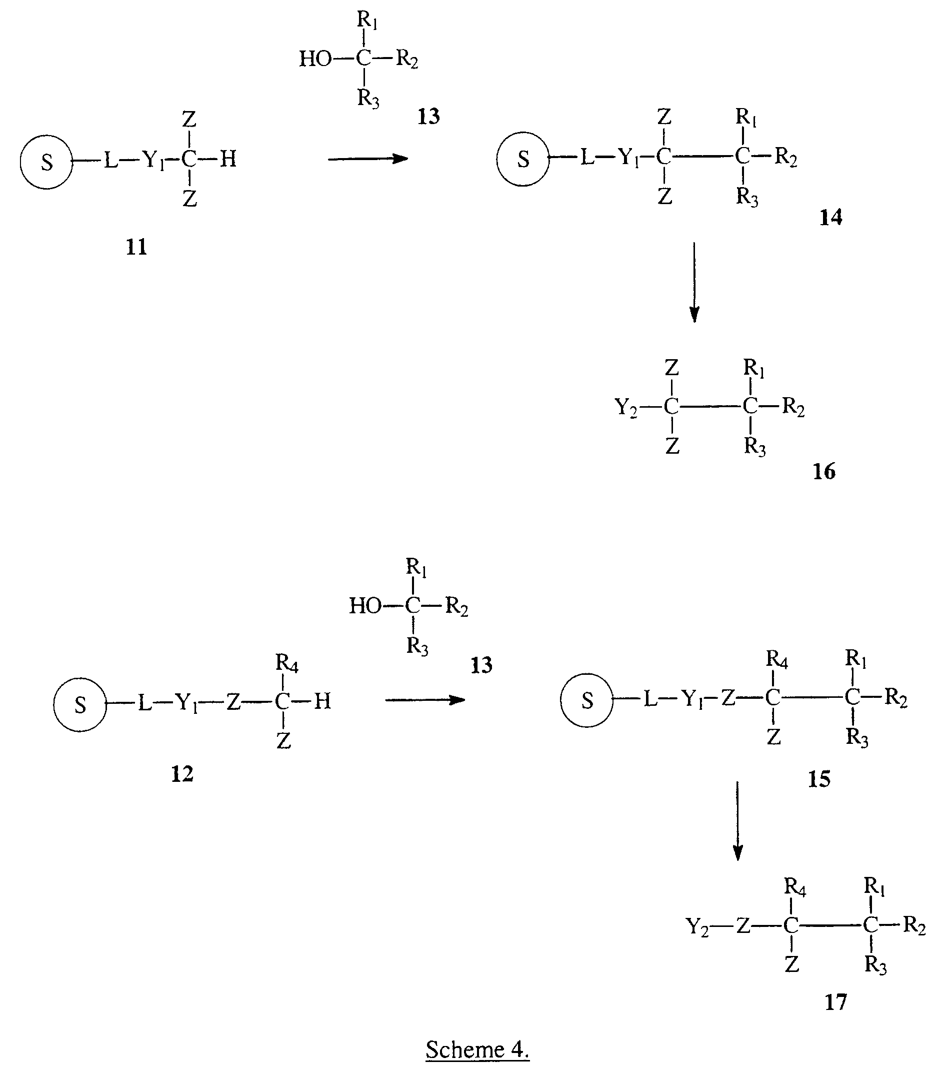

An embodiment of the present invention is a process for forming a carbon-carbon bond between a solid support-bound hydroxyl reagent **7** and an active methylene reagent **8,** where Z are electron-withdrawing substituents, such as carboxylic acid, ester, amide, alkylketone, arylketone, aldehyde, cyano, aldehyde, alkylsulfone, arylsulfone, sulfoxide, sulfonamide, sulfonate, nitro, aryl, phosphonate, and phosphate and the like, and R₁ is H, lower alkyl, aryl, and halogen substituents, with a trivalent phosphorus reagent **1** and an azo-containing reagent **2** to form Mitsunobu alkylation product **9**, according to Scheme 3.

Another embodiment of the invention includes processes for loading solid supports with compounds to prepare solid support-bound active methylene reagents. As for functionalized support **7,** support **6** is loaded with reagents to provide active methylene supports **11** and **12** (Scheme 4). Processes are provided for forming carbon-carbon bonds between **11** and **12** and a hydroxyl reagent **13** by the Mitsunobu alkylation where the reactants are reversed relative to that in Scheme 3 to yield products **14** and **15,** respectively.

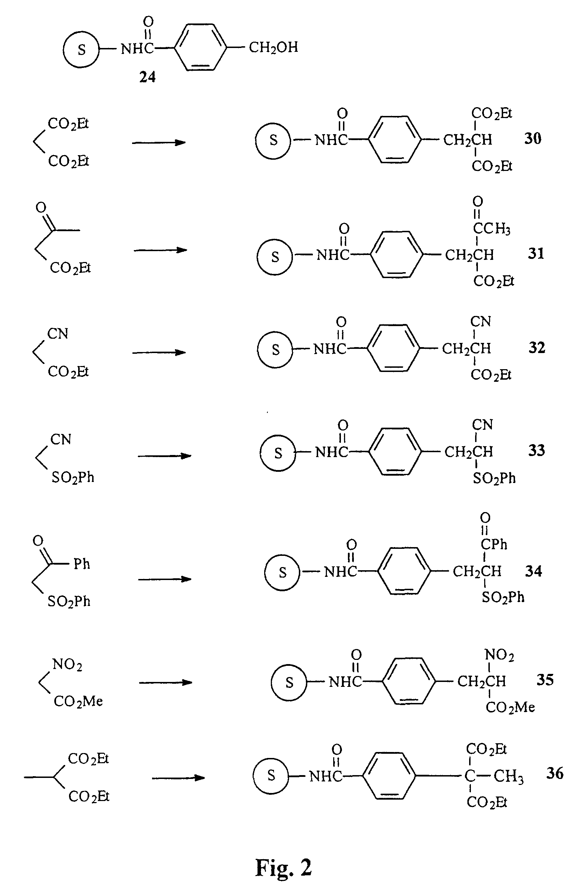

An azo-containing compound and a trivalent phosphorous compound are reagents used in the Mitsunobu alkylation reaction to activate the hydroxyl functionality of one of the reactants. Any azo-containing compound and any trivalent phosphorous compound capable of leading to the Mitsunobu alkylation reaction product may be employed. Azo-containing compounds contain the Z-N=N-Z functional moiety. Preferred compounds include tetramethylazodicarboxamide (TMAD), diethylazodicarboxylate (DEAD), diisopropylazodicarboxylate, and 1,1'-(azodicarbonyl)dipiperidine (Tsunoda, 1994). Trivalent phosphorus compounds have three substituents R in the general structure, PR₃. Substituents R include lower alkyl, phenyl, aryl, lower alkoxy, phenoxy, and aryloxy. Preferred compounds include tributylphosphine, triethylphosphine, and triphenylphosphine. A particularly preferred combination is TMAD and tributyl phosphine (Ito, 1994). The Mitsunobu alkylation reaction of the present invention can be conducted in any solvent capable of solubilizing the reagents, other than the support. Preferred solvents include tetrahydrofuran (THF), dimethoxyethane (DME), dichloromethane, dimethylformamide, acetonitrile, and other non-protic solvents. Compounds **30-36** synthesized by Mitsunobu alkylation are illustrated in Figure 2 and Examples 8-14.

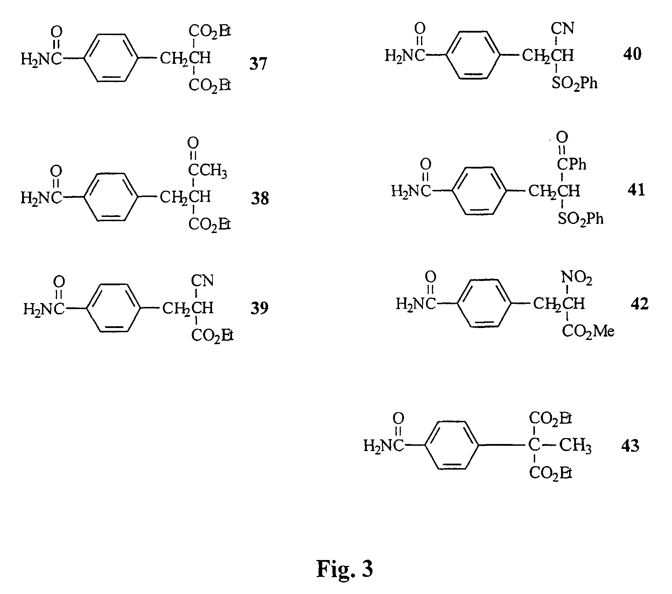

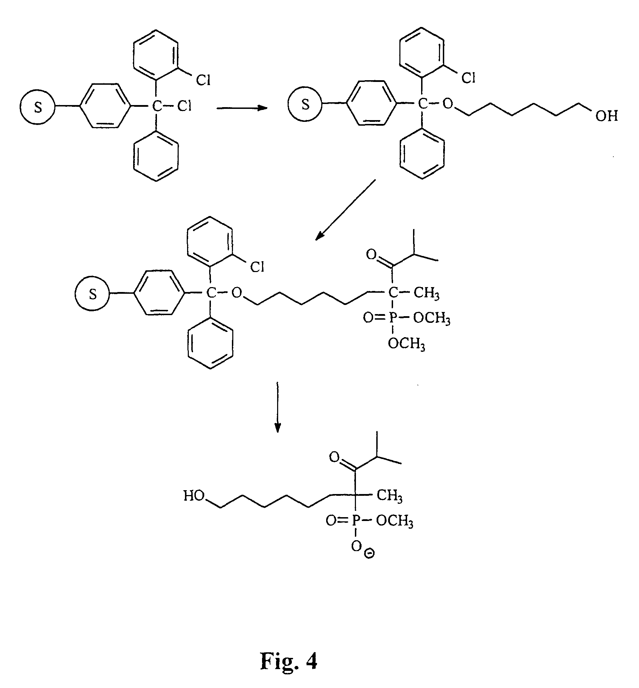

The products of solid support Mitsunobu alkylation may be cleaved from the solid supports **9, 14,** and **15** with acidic, basic, or specific cleavage reagents, depending on the nature of the linker, L, and functionality bonded to the linker, Y₁ to give cleaved products **10, 16,** and **17,** respectively, where Y₂ is a group resulting from cleavage of the linker and Y₁, possess a broad variety of molecular diversity and utility (Schemes 2 and 3). Specific examples of cleaved products are illustrated by **37-43** in Figure 3 and Examples 16-22. Rink and Wang type linkers attached to Merrifield polystyrene (Scheme 2), commonly used in peptide synthesis to prepare carboxyl and carboxamide terminus peptides, respectively, are cleaved under moderate to strong acid conditions, e.g. 90% trifluoroacetic acid. Trityl linkers, such as 2-chlorotrityl, require briefer and milder acid conditions, e.g. 30% acetic acid (Figure 4 and Example 23). Cation scavengers are sometimes added to the cleavage mixture to minimize by-products from sensitive functionality. Linkers which form a base-labile linkage can be cleaved with hydroxide, ammonia, alkylamine, and other basic reagents. Alkoxysilane linkages can be selectively cleaved under anhydrous and mild conditions with fluoride ion (Greene, 1991). A typical reagent for cleavage of trialkylsilylether linkages is tetra n-butylammonium fluoride in tetrahydrofuran (Aldrich Chemical Co., Milwaukee, WI).

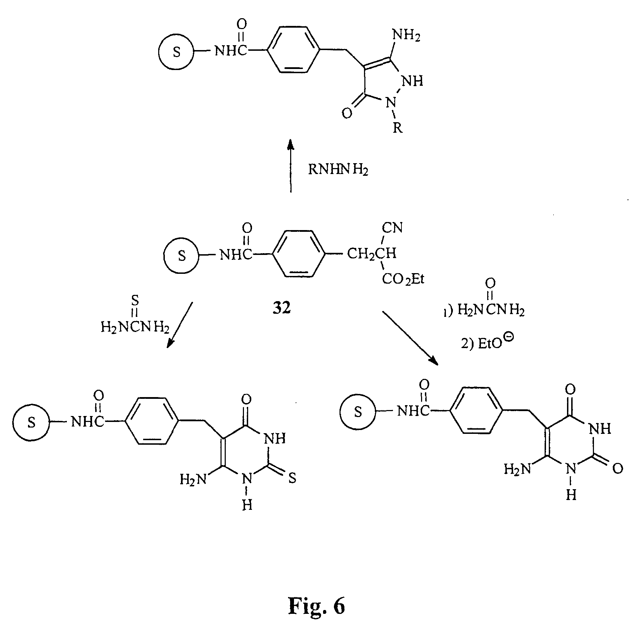

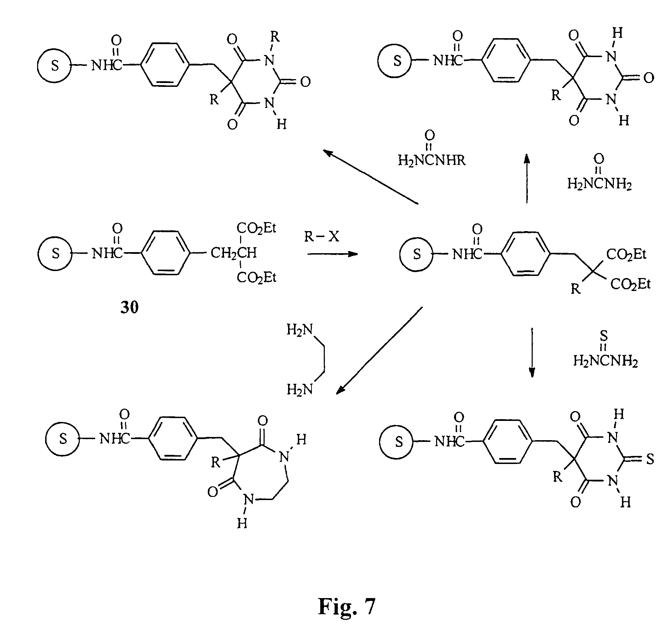

The products from solid Mitsunobu alkylation are extremely useful and versatile intermediates, easily converted to heterocyclic compounds with known biological activity. For example, 1,3-ketoesters, e.g. **31** (Figure 3) cyclize with alkylhydrazines to give pyrazoles, a class of compounds with anticonvulsant activity (Saxena, 1995). Further alkylation of the support-bound product on nitrogen increases the diversity potential. Also, 1,3-ketonitriles react with alkylhydrazine, urea, and thiourea reagents to form 5-aminopyrazoles, 5-aminopyrimidines, and 5-amino-2-thiopyrimidines, respectively (Watson, 1997), as illustrated by cyclization reactions of **32** (Figure 6). Additionally, *O*-nitroarylacetonitriles, reported as excellent active methylene compounds for Mitsunobu alkylation (Macor, 1991), lead to a wide variety of indole and tryptophan derivatives after nitro reduction and cyclization. Figure 7 shows 1,3-diester compounds, resulting from Mitsunobu alkylation, such as **30**, can be alkylated and cyclized to form 6- and 7-membered rings, yielding barbiturate- and diazepam-type structures. Mitsunobu alkylation products on the support, such as **30-36,** or cleaved in solution, such as **37-43,** can be further reacted with other reagents and converted to compounds with antipyretic, barbituric, and antimicrobial activity (Katsuyama, 1997).

The Mitsunobu alkylation reaction on solid support can be applied to the combinatorial chemistry method for synthesis of large numbers of compounds (Houghten, 1991). Compounds can be made rapidly in parallel processes, rather than by sequential reiteration of individual syntheses. As a versatile and efficient chemical reaction, the Mitsunobu alkylation can be conducted with a large number of reagents with similar or identical functionality and reactivity. When activity is noted, then active species are identified, either by positional deconvolution or an encoded tag scheme (Baldwin, 1998; Brenner, 1992). A very high level of diversity of shapes, sizes, charge, and other physical parameters, can be synthesized quickly and submitted to high-throughput screening assays (Baldwin, 1998), such as enzyme inhibitor assays (Baldwin, 1997). When the library of compounds is made on beads or other loose particles, they may be manipulated spatially. When at any time during a synthesis process, the support is combined and redistributed, this "split and mix" routine generates a higher level of structural diversity of heterogeneous mixtures of compounds (Gallop, 1996; Dower, 1997). The present invention may be practiced on devices that automate some or all of the chemistry and/or manipulations with robotic arms, liquid dispensing units, and inert gas enclosures for delivery of reagents and movement of the solid supports on a computer-directed, work surface array. Examples 24 and 25 the synthesis of libraries of compounds by parallel and the "split and mix" approach, respectively. In these experiments, the solid-phase Mitsunobu alkylation reaction provides molecular diversity in the production of many compounds due to its efficiency and versatility.

In addition to beads and particles, high-throughput parallel synthesis of compounds by the Mitsunobu alkylation reaction can be conducted on supports with surfaces which have been derivatized with functionality by chemical treatment, radioisotope bombardment, e.g. ⁶⁰Co, or other high-energy radical treatment. Examples of inorganic surfaces include silicon wafers, glass, silica, and alumina. Examples of organic surfaces include polyethylene, polypropylene, polyacrylates, polyacrylamides, polystyrene, polyfluoroethylene, latex, cellulose, polyethylene glycol, or grafts, co-polymers, and composites of such. A derivatized solid surface may bear a large number and high-density array of reaction sites, which may be individually addressed by delivering different or common reagents by robotic liquid dispensing apparatus or by ink-jet, micro-droplet spraying technology. The reaction sites of a two-dimensional array may yield 10-1000, or more, compounds segregated in a grid-like configuration, often termed a "library". The beads may be collected from their reaction sites, mixed, redistributed, and further reacted. In this manner, heterogeneous mixtures of uncharacterized compounds are synthesized. Iterating this "split and mix" method yields a large number of compounds, represented by extensive molecular diversity of structure, shape, and other properties. The compounds may be cleaved and collected individually or pooled. Alternatively, the compounds may remain on the solid support and undergo heterogeneous assay. An advantage of the "split and mix" method is that each bead contains only one compound, allowing the compounds to be screened on the beads themselves. However, the identity of the compounds on each bead must be independently determined, often by a tagging scheme whereby compounds more readily analyzable than the library themselves are selectively attached to the beads (Brenner, 1992).

Solid supports can be in the form of beads of 1-1000 µm diameter, pellets, disks, wafers, frits, and planar or irregular surfaces. The preferred embodiment of the solid support for the present invention is microporous polystyrene beads, cross-linked with 1-2% of divinylbenzene. Beads, and other loose particles, of the solid synthesis supports may also be configured in an array configuration whereby they reside in wells, chambers, or columns addressable by liquid dispensing apparatus. A common dimensional format is the 96 well microtiter plate.

### EXAMPLES

The invention will be further clarified by a consideration of the following examples, which are intended to be purely exemplary of the present invention and not to in any way limit its scope.

### General Experimental Conditions and Abbreviations:

Syntheses of compounds on solid-supports were conducted on the Solaris™ 530 Organic Synthesizer (PE Biosystems, Foster City, CA). HPLC analyses were performed using a Perkin-Elmer (Norwalk, CT) Series 200 IC pump at 1 ml/min flow, Model 235C diode array detector monitoring at 255 nm and 355 nm, Targa column (C18, 5µm, 50x46 mm) with isocratic elution in 5% acetonitrile (ACN) in 0.1 M TEAA, pH=7.0, and sample injection size of 20 µl. NMR analyses were performed on a Varian Unity 300 spectrometer, ¹H resonances detected at 300MHz. Mass Spectroscopy mass analyses were performed on a PE-Sciex API-100 single-quadrupole (Perkin-Elmer, Norwalk, CT) operating in heated nebulizer mode with corona discharge and positive ion APCI, at 400 °C. Positive-ion mode scanning, m/z 50-600, step at 0.25 amu, dwell 1.0 ms.

### Solid supports and linkers:

Rink amide: 4-(2',4'-dimethoxyphenyl-Fmoc-aminomethyl)-phenoxy copolystyrene-di vinyl benzene (DVB)
Wang: p-benzyloxybenzyl alcohol copolystyrene-DVB
Merrifield: chloromethylated copolystyrene-DVB
PAM: 4-hydroxymethyl-phenylacetamidomethyl
MBHA: p-methylbenzhydrylamine
Tenta gel: composite of polyethylene oxide grafted onto a low cross-linked polystyrene gel-type matrix

### EXAMPLE 1

### Synthesis of polystyrene-Rink linker-hydroxymethylbenzamide 24 by loading of hydroxymethylbenzoic acid 18 onto polystyrene-Rink linker (Figure 1)

In a sintered glass filter vessel connected to vacuum from below and a pressure release and argon atmosphere above, 2.00 gm of 4-(2',4'-dimethoxyphenylfluorenylmethoxycarbonylaminomethyl)phenoxy polystyrene, "Rink amide resin" (Rink, 1987), loaded at 0.6 mmol Fmoc-amine/gm was subjected to five washes with 15 ml of 20% piperidine in dimethylformamide (DMF), whereupon no UV absorption from Fmoc was detected in the eluate. A 0.3M solution (22 ml) of hydroxymethylbenzoic acid **18** (3 equivalents), 3.3 equivalents diisopropylcarbodiimide (DICD) and 3.3 equivalents of N-hydroxybenzotriazole (HOBt) in DMF at room temperature was reacted at room temperature for 40 minutes to preactivate the acid. The activated acid solution was added to the polystyrene-Rink linker amine in the vessel and the vessel was agitated by a wrist-action shaker. The coupling was complete in four hours, when the Kaiser test for free amine on a small sample of support was negative (Kaiser, 1970). The reagents were removed by filtration under vacuum and the support, washed successively with 20 ml portions of DMF, ethanol, and diethylether, and dried under vacuum to give 1.95 gm of polystyrene-Rink linker-hydroxymethylbenzamide **24**.

### EXAMPLE 2

### Synthesis of polystyrene-Rink linker-4-hydroxymethyl-3-methoxyphenoxyacetamide 24 by loading of 4-hydroxymethyl-3-methoxyphenoxyacetic acid 19 onto polystyrene-Rink linker (Figure 1)

Using the conditions and reagent proportions of Example 1, the above support **24** is obtained.

### EXAMPLE 3

### Synthesis of polystyrene-Rink linker-4-(4-hydroxymethyl-3-methoxyphenoxy)butyramide 26 by loading of 4-(4-hydroxymethyl-3-methoxyphenoxy) butyric acid 20 onto polystyrene-Rink linker (Figure 1)

Using the conditions and reagent proportions of Example 1, the above support **26** is obtained.

### EXAMPLE 4

### Synthesis of polystyrene-Rink linker-4-(hydroxymethyl)phenoxyacetamide 27 by loading of 4-(hydroxymethyl)phenoxyacetic acid 21 onto polystyrene-Rink linker (Figure 1)

Using the conditions and reagent proportions of Example 1, the above support **27** is obtained.

### EXAMPLE 5

### Synthesis of polystyrene-Rink linker-3-(4-hydroxymethylphenoxy)propionamide 28 by loading of 3-(4-hydroxymethylphenoxy)propionic acid 22 onto polystyrene-Rink linker (Figure 1)

Using the conditions and reagent proportions of Example 1, the above support **28** is obtained.

### EXAMPLE 6

### Synthesis of polystyrene-Rink linker-5-(4-hydroxymethyl-3-methoxyphenoxy) valeramide 29 by loading of 5-(4-hydroxymethyl-3-methoxyphenoxy)valeric acid 23 onto polystyrene-Rink linker (Figure 1)

Using the conditions of Example 1, the above support **29** is obtained.

### EXAMPLE 7

### Synthesis of polystyrene/polyethylene glycol-hydroxymethylbenzamide by loading of hydroxymethylbenzoic acid 18

Low cross-link polystyrene is grafted with polyethylene glycol (PEG), by polymerization with ethylene oxide. The PEG chains are terminated with amino Fmoc functionality (TentaGel S RAM Fmoc, available from Rapp Polymere). Deprotection of the Fmoc group with 20% piperidine in DMF generates free amine. The resin is washed with DMF, methanol, and dichloromethane in a fritted funnel under vacuum. Coupling with 4-hydroxymethylbenzoic acid can give polystyrene/polyethylene glycol-hydroxymethylbenzamide (Rano, 1995).

### EXAMPLE 8

### Synthesis of polystyrene-Rink linker amide of diethyl 2-(4-carbamoylphenyl) propane-1,3-dioate 30 (Figure 2)

Polystyrene-Rink linker-hydroxymethylbenzamide **24** (1.00 gm, 1.1 mmol OH/gm) was swollen in 15 ml tetrahydrofuran (THF) for 10 minutes under an argon atmosphere at room temperature in a sintered glass filter reaction vessel with inlet and exit ports. Tributylphosphine (1.37 ml, 5.5 mmol), tetramethylazodicarboxamide (0.95 gm in 5 ml THF, 5.5 mmol), and diethyl malonate (0.84 ml, 5.5 mmol) were added successively by syringe through the inlet port. After shaking for 12 hours, reagents were removed by filtration under a slight positive pressure of argon, followed by washing with 25 ml portions of DMF, methanol, and dichloromethane. The product support **30** was obtained after drying under vacuum as an off-white powder.

### EXAMPLE 9

### Synthesis of polystyrene-Rink linker amide of ethyl 2-(4-carbamoylphenyl)-3-oxobutanoate 31 (Figure 2)

Polystyrene-Rink linker-hydroxymethylbenzamide **24** (1.00 gm, 1.1 mmol OH/gm) was swollen in 15 ml THF for 10 minutes under an argon atmosphere at room temperature in a sintered glass filter reaction vessel with inlet and exit ports. Triphenylphosphine (1.44 gm, 5.5 mmol), tetramethylazodicarboxamide (0.95 gm in 5 ml THF, 5.5 mmol), and ethyl acetoacetate (0.70 ml, 5.5 mmol) were added successively by syringe through the inlet port. After shaking for 12 hours, reagents were removed by filtration under a slight positive pressure of argon, followed by washing with 25 ml portions of DMF, methanol, and dichloromethane. The product support **31** was obtained after drying under vacuum as an off-white powder.

### EXAMPLE 10

### Synthesis of polystyrene-Rink linker amide of ethyl 2-(4-carbamoylphenyl)-2-cyanoacetate 32 (Figure 2)

Polystyrene-Rink linker-hydroxymethylbenzamide **24** (1.00 gm, 1.1 mmol OH/gm) was swollen in 15 ml THF for 10 minutes under an argon atmosphere at room temperature in a sintered glass filter reaction vessel with inlet and exit ports. Tributylphosphine (1.37 ml, 5.5 mmol), diethylazodicarboxylate (0.87 ml, 5.5 mmol), and ethyl cyanoacetate (0.59 ml, 5.5 mmol) were added successively by syringe through the inlet port. After shaking for 12 hours, reagents were removed by filtration under a slight positive pressure of argon, followed by washing with 25 ml portions of THF, ethanol, and diethylether. The product support **32** was obtained after drying under vacuum as an off-white powder.

### EXAMPLE 11

### Synthesis of polystyrene-Rink linker amide of 4-[cyano(phenylsulfonyl) methyl]benzamide 33 (Figure 2)

Polystyrene-Rink linker-hydroxymethylbenzamide **24** (1.00 gm, 1.1 mmol OH/gm) was swollen in 15 ml THF for 10 minutes under an argon atmosphere at room temperature in a sintered glass filter reaction vessel with inlet and exit ports. Tributylphosphine (1.37 ml, 5.5 mmol), 1,1'-(azodicarbonyl)dipiperidine (1.39 gm in 5 ml THF, 5.5 mmol), and (phenylsulfonyl)acetonitrile (1.00 gm in 5 ml THF, 5.5 mmol) were added successively by syringe through the inlet port. After shaking for 12 hours, reagents were removed by filtration under a slight positive pressure of argon, followed by washing with 25 ml portions of THF, ethanol, and diethylether. The product support **33** was obtained after drying under vacuum as an off-white powder.

### EXAMPLE 12

### Synthesis of polystyrene-Rink linker amide of 4-[2-oxo-2-phenyl-1-(phenylsulfonyl) ethyl]benzamide 34 (Figure 2)

Polystyrene-Rink linker-hydroxymethylbenzamide **24** (1.00 gm, 1.1 mmol OH/gm) was swollen in 15 ml 1,2-dimethoxyethane (DME) for 10 minutes under an argon atmosphere at room temperature in a sintered glass filter reaction vessel with inlet and exit ports. Tributylphosphine (1.37 ml, 5.5 mmol), tetramethylazodicarboxamide (0.95 gm in 5 ml THF, 5.5 mmol) and 2-(Phenylsulfonyl)acetophenone (1.43 gm in 5 ml DME, 5.5 mmol) were added successively by syringe through the inlet port. After shaking for 12 hours, reagents were removed by filtration under a slight positive pressure of argon, followed by washing with 25 ml portions of DME, ethanol, and diethylether. The product support **34** was obtained after drying under vacuum as an off-white powder.

### EXAMPLE 13

### Synthesis of polystyrene-Rink linker amide of methyl 2-(4-carbamoylphenyl)-2-nitroacetate 35 (Figure 2)

Polystyrene-Rink linker-hydroxymethylbenzamide **24** (1.00 gm, 1.1 mmol OH/gm) was swollen in 15 ml DME for 10 minutes under an argon atmosphere at room temperature in a sintered glass filter reaction vessel with inlet and exit ports. Tributylphosphine (1.37 ml, 5.5 mmol), tetramethylazodicarboxamide (0.95 gm in 5 ml DME, 5.5 mmol), and methyl nitroacetate (0.51 gm, 5.5 mmol) were added successively by syringe through the inlet port. After shaking for 12 hours, reagents were removed by filtration under a slight positive pressure of argon, followed by washing with 25 ml portions of DME, ethanol, and diethylether. The product support **29** was obtained after drying under vacuum as an off-white powder.

### EXAMPLE 14

### Synthesis of polystyrene-Rink linker amide of diethyl 2-(4-carbamoylphenyl), 2-methyl propane-1,3-dioate 36 (Figure 2)

Polystyrene-Rink linker-hydroxymethylbenzamide **24** (1.00 gm, 1.1 mmol OH/gm) was swollen in 15 ml tetrahydrofuran (THF) for 10 minutes under an argon atmosphere at room temperature in a sintered glass filter reaction vessel with inlet and exit ports. Tributylphosphine (1.37 ml, 5.5 mmol), tetramethylazodicarboxamide (0.95 gm in 5 ml THF, 5.5 mmol), and diethyl methyl malonate (0.95 ml, 5.5 mmol) were added successively by syringe through the inlet port. After shaking for 12 hours, reagents were removed by filtration under a slight positive pressure of argon, followed by washing with 25 ml portions of DMF, methanol, and dichloromethane. The product support **36** was obtained after drying under vacuum as an off-white powder.

### EXAMPLE 15

### Synthesis of polystyrene-Rink linker-hydroxymethylbenzamide 24 by loading of 4-carboxybenzaldehyde and reduction

In a sintered glass filter vessel connected to vacuum from below and a pressure release and argon atmosphere above, 2.00 gm of 4-(2',4'-dimethoxyphenylfluorenylmethoxycarbonylaminomethyl)phenoxy polystyrene, "Rink amide resin" (Rink, 1987), loaded at 0.64 mmol Fmoc-amine/gm is subjected to five washes with 15 ml of 20% piperidine in dimethylformamide (DMF), whereupon no UV absorption from Fmoc may be detected in the eluate. A 0.3M solution (22 ml) of 4-carboxybenzaldehyde (3 equivalents), 3.3 equivalents diisopropylcarbodiimide (DICD) and 3.3 equivalents of N-hydroxybenzotriazole (HOBt) in DMF at room temperature may be reacted at room temperature for 40 minutes to preactivate the acid. The activated acid solution is added to the polystyrene-Rink linker amine in the vessel and the vessel is agitated by a wrist-action shaker. The coupling is complete in two hours, when the Kaiser test for free amine on a small sample of support is negative (Kaiser, 1970). The reagents are removed by filtration under vacuum and the support, washed successively with 20 ml portions of DMF, ethanol, and diethylether, and dried under vacuum to give 1.95 gm of polystyrene-Rink linker-(benzamide-4-carboxaldehye). The support is swollen in THF and stirred with 10 equivalents NaBH₄ in 20 ml MeOH:DCM:THF / 0.5:1:1 for 12 hr. The solvent was aspirated off and the reduction was continued for another 12 hr using fresh reagent and solvent. After 12 hr, the solvent was removed and the support was sequentially washed with 10% aqueous mannitol, water, MeOH, and DCM (each 3 x 10 ml).

### EXAMPLE 16

### Synthesis of diethyl 2-(4-carbamoylphenyl) propane-1,3-dioate 37 by cleavage of polystyrene-Rink linker amide of diethyl 2-(4-carbamoylphenyl) propane-1,3-dioate 30 (Figure 3)

Support **30** (1.0 gm) is treated with 10 ml of a mixture of TFA:dichloromethane (DCM) / 1:4 and shaken for 1 hour at room temperature under an argon atmosphere. The cleavage mixture liquid was drained and collected. The support is washed with 5 ml THF and the wash is combined with the cleavage mixture and rotary evaporated under reduced pressure. The residue is taken up in 10 ml ethyl acetate and evaporated to give diethyl 2-(4-carbamoylphenyl)propane-1,3-dioate **37** (0.307 gm), identified by ¹H NMR: (CDCl₃ δ 7.28 (d, 2Hₐᵣ, J=8.1), 7.75 (d, 2Hₐᵣ, J=8.1), 4.2 (m, -CH₂), 3.65 (t, CH₂), 1.2 (t, CH₃) and mass spectroscopy: 294.3, M+1, and assessed for purity by HPLC: 12.1 minute retention time.

### EXAMPLE 17

### Synthesis of ethyl 2-(4-carbamoylphenyl)-3-oxobutanoate 38 by cleavage of polystyrene-Rink linker amide of ethyl 2-(4-carbamoylphenyl)-3-oxobutanoate 31 (Figure 3)

Support **31** (1.0 gm) is treated with 10 ml of a mixture of TFA:dichloromethane (DCM) / 1:4 for 1 hour at room temperature under an argon atmosphere. The cleavage mixture liquid was drained and collected. The support is washed with 5 ml THF and the wash is combined with the cleavage mixture and rotary evaporated under reduced pressure. The residue is taken up in 10 ml ethyl acetate and evaporated to give ethyl 2-(4-carbamoylphenyl)-3-oxobutanoate **38** (0.274 gm), identified by ¹H NMR: (CDCl₃ δ 7.3 (d, 2Hₐᵣ, J=8.0), 7.70 (d, 2Hₐᵣ, J=8.0), 4.18 (q, -CH₂), 3.85 (t, -CH), 3.2 (m, -CH₂), 2.2 (s, -CH₃), 1.2 (t, CH₃) and mass spectroscopy: 264.3, M+1, and assessed for purity by HPLC: 11.9 minute retention time.

### EXAMPLE 18

### Synthesis of ethyl 2-(4-carbamoylphenyl)-2-cyanoacetate 39 by cleavage of polystyrene-Rink linker amide of ethyl 2-(4-carbamoylphenyl)-2-cyanoacetate 32 (Figure 3)

Support **32** (1.0 gm) is treated with 10 ml of a mixture of TFA:dichloromethane (DCM) / 1:4 for 1 hour at room temperature under an argon atmosphere. The cleavage mixture liquid was drained and collected. The support is washed with 5 ml THF and the wash is combined with the cleavage mixture and rotary evaporated under reduced pressure. The residue is taken up in 10 ml ethyl acetate and evaporated to give ethyl 2-(4-carbamoylphenyl)-2-cyanoacetate **39** (0.255 gm), identified by ¹H NMR and mass spectroscopy: 247.3, M+1, and assessed for purity by HPLC: 11.2 minute retention time.

### EXAMPLE 19

### Synthesis of 4-[cyano(phenylsulfonyl) methyl]benzamide 40 by cleavage of polystyrene-Rink linker amide of 4-[cyano(phenylsulfonyl) methyl]benzamide 33 (Figure 3)

Support **33** (1.0 gm) is treated with 10 ml of a mixture of TFA:dichloromethane (DCM) / 1:4 for 1 hour at room temperature under an argon atmosphere. The cleavage mixture liquid was drained and collected. The support is washed with 5 ml THF and the wash is combined with the cleavage mixture and rotary evaporated under reduced pressure. The residue is taken up in 10 ml ethyl acetate and evaporated to give 4-[cyano(phenylsulfonyl) methyl]benzamide **40** (0.330 gm), identified by ¹H NMR: (CDCl₃ δ 7.38 (d, 2Hₐᵣ, J=8.1), 8.12 (d, 2Hₐᵣ, J=8.1), 7.61-7.85 (5H, Ar), 4.12 (dd, 1H, J=4.1, 11.75), 3.65 (dd, 1H, J=4.1, 13), 3.2 (dd, 1H, J=2, 13.58) and mass spectroscopy: 315.3, M+1, and assessed for purity by HPLC: 10.47 minute retention time.

### EXAMPLE 20

### Synthesis of 4-[2-oxo-2-phenyl-1-(phenylsulfonyl) ethyl]benzamide 41 by cleavage of polystyrene-Rink linker amide of 4-[2-oxo-2-phenyl-1-(phenylsulfonyl) ethyl]benzamide 34 (Figure 3)

Support **34** (1.0 gm) is treated with 10 ml of a mixture of TFA:dichloromethane (DCM) / 1:4 for 1 hour at room temperature under an argon atmosphere. The cleavage mixture liquid was drained and collected. The support is washed with 5 ml THF and the wash is combined with the cleavage mixture and rotary evaporated under reduced pressure. The residue is taken up in 10 ml ethyl acetate and evaporated to give 4-[2-oxo-2-phenyl-1-(phenylsulfonyl) ethyl]benzamide **41** (0.417 gm), identified by ¹H NMR and mass spectroscopy: 395, M+1, and assessed for purity by HPLC: 11.82 minute retention time.

### EXAMPLE 21

### Synthesis of methyl 2-(4-carbamoylphenyl)-2-nitroacetate 42 by cleavage of polystyrene-Rink linker amide of methyl 2-(4-carbamoylphenyl)-2-nitroacetate 35 (Figure 3)

Support **35** (1.0 gm) is treated with 10 ml of a mixture of TFA:dichloromethane (DCM) / 1:4 for 1 hour at room temperature under an argon atmosphere. The cleavage mixture liquid was drained and collected. The support is washed with 5 ml THF and the wash is combined with the cleavage mixture and rotary evaporated under reduced pressure. The residue is taken up in 10 ml ethyl acetate and evaporated to give methyl 2-(4-carbamoylphenyl)-2-nitroacetate **42** (0.262 gm), identified by ¹H NMR and mass spectroscopy.

### EXAMPLE 22

### Synthesis of diethyl 2-(4-carbamoylphenyl), 2-methyl propane-1,3-dioate 43 by cleavage of polystyrene-Rink linker amide of diethyl 2-(4-carbamoylphenyl), 2-methyl propane-1,3-dioate 36 (Figure 3)

Support **36** (1.0 gm) is treated with 10 ml of a mixture of TFA:dichloromethane (DCM) / 1:4 and shaken for 1 hour at room temperature under an argon atmosphere. The cleavage mixture liquid was drained and collected. The support is washed with 5 ml THF and the wash is combined with the cleavage mixture and rotary evaporated under reduced pressure. The residue is taken up in 10 ml ethyl acetate and evaporated to give diethyl 2-(4-carbamoylphenyl), 2-methyl propane-1,3-dioate **43** (0.321 gm), identified by ¹H NMR: (CDCl₃ δ 7.26 (d, 2Hₐᵣ, J=8.0), 7.76 (d, 2Hₐᵣ, J=8.0), 4.2 (q, -CH₃), 3.32 (s, CH₂), 1.36 (s, CH₃), 1.23 (t, -CH₃) and mass spectroscopy: 308.3, M+1, and assessed for purity by HPLC: 14.5 minute retention time.

### EXAMPLE 23

### Synthesis of 11-hydroxy-4-(methoxy(hydroxyphosphoryl))-2,4-dimethylundecan-3-one (Figure 4)

In a sintered glass filter vessel connected to vacuum from below and a pressure release and argon atmosphere above, 2.00 gm 2-chlorotritylchloride-polystyrene, loaded at 1.5 mmol chloride/gm is swollen in 30 ml dry pyridine. 1,6-Hexanediol (3.54 gm, 0.03 mol) is added and the reaction is shaken at room temperature for 4 hours. The reagents were removed by filtration under vacuum, the support was washed with 4x20 ml portions of DCM, and dried under vacuum. After swelling with 20 ml 1,2-dimethoxyethane (DME), 4-dimethoxyphosphoryl-2-methylpentan-3-one (3.12 gm, 0.015 mol), tributylphosphine (3.7 ml, 0.015 mol), and tetramethylazodicarboxamide (2.59 gm in 15 ml DME, 0.015 mol) were added successively by syringe through the inlet port. After shaking for 12 hour, reagents are removed by filtration under a slight positive pressure of argon, washed with 25 ml portions of DME, ethanol, and diethylether, and dried under vacuum. The support is treated with 25 ml of acetic acid:DCM / 1:4 for 1 hr at room temperature. The cleavage mixture liquid was drained and collected. The support is washed with 5 ml DCM and the wash is combined with the cleavage mixture and rotary evaporated under preduced pressure. The residue is taken up in 10 ml ethyl acetate and evaporated to give 11-hydroxy-4-(methoxy(hydroxyphosphoryl))-2,4-dimethylundecan-3-one (0.79 gm) identifed by ¹H NMR and mass spectroscopy.

### EXAMPLE 24

### Synthesis of an array

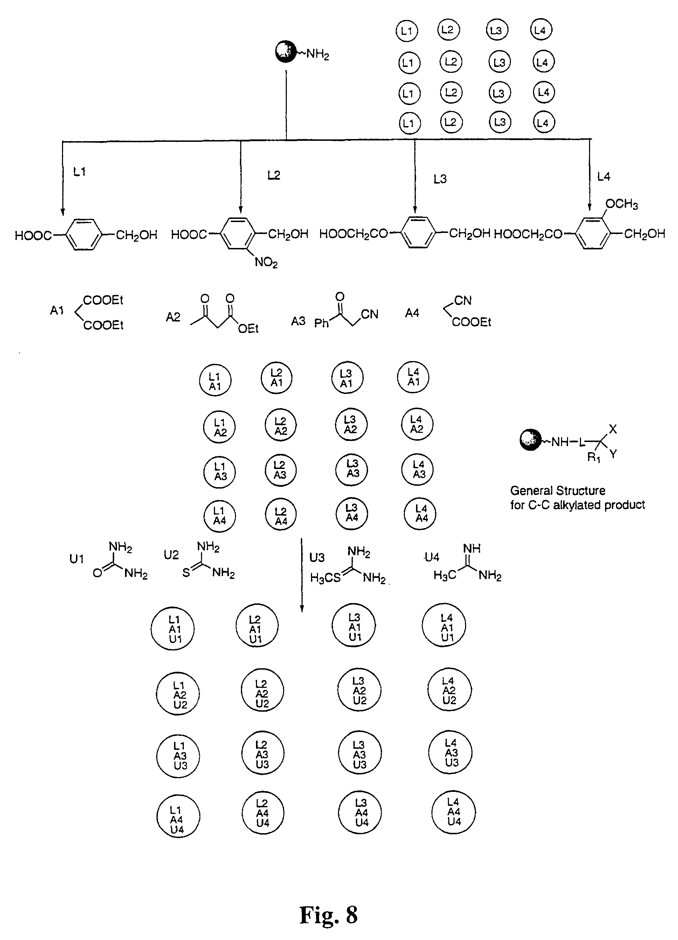

The synthesis of sixteen biologically active compounds with anticonvulsant activity is performed in a parallel array on a robotic liquid handler synthesizer (Solaris™ 530 Organic Synthesizer). The additions, aspirations, and washings are performed using a robotic arm possessing four alternate aspirating and coaxial tips. Fifty milligrams of the Rink Amide resin, loaded at 0.6 mmol Fmoc-amine/gm, is placed in each of 16 round-bottomed flasks in a 4x4 array (Figure 8). The Fmoc groups are cleaved with 20% piperidine in DMF for 10 min. The support is subjected to a series of washing (3x5 ml) with each of DMF, MeOH, and DCM. Four carboxylic acid-hydroxyl reagents (L1-L4) delivered to four reaction vessels each and coupled to the support using 3 equivalents of DIC and HOBt in DMA for 4 hr. Excess liquid reagents are drained and each flask is subjected to standard washing routine and drying under vacuum. The next step is the Mitsunobu C-C alkylation with four different active methylene compounds (A1-A4) and five equivalents each of tributyl phosphine and TMAD in THF at room temperature. In particular, A1 is delivered to flasks with L1, L2, L3, and L4. In like manner, A2 is delivered to the next row of L1-L4. And so on with A3 and A4 to create a 16 flask array of unique compounds. Tributyl phosphine and TMAD in THF are delivered to each of the 16 flasks. After 12hr of shaking at room temperature, excess liquid reagents are drained and the resin is subjected to washing and drying. Finally, the cyclization reaction is conducted with four urea derivatives (U1-U4) in refluxing sodium ethoxide in THF/EtOH. As in the delivery of A1-A4, U1-U4 are delivered to create 16 unique compounds in the 16 flask array. The products cleaved from the support with 20% TFA in DCM gives the 16 anticonvulsant compounds.

### EXAMPLE 25

### Synthesis of an array by combining and distributing supports (Figure 9).

Figure 9 illustrates the synthesis of 64 biologically active antipyretic compounds in a 16 reaction vessel array. The synthesis protocol is similar to what is described in the parallel approach (Example 24, Figure 8). However, in this example, after each transformation the support from each flask is combined, thoroughly mixed, and divided (distributed) into equal-sized portions in each of the 16 reaction vessels. Fifty milligrams of polystyrene-Rink amide, loaded at 0.6 mmol Fmoc-amine/gm, is placed in each of 16 round bottomed flasks in a 4x4 array and subjected to Fmoc cleavage with 20% piperidine in DMF for 10 min. The support is then washed (3x5 ml) with DMF, MeOH, and DCM. Various carboxylic acid-hydroxyl reagents (L1-L4) are coupled to the amino support using 3 equivalents each of DIC and HOBt in DMA for 4 hr. Each of the four reagents, L1-L4, are delivered to a row of four flasks. The solvent is drained and each flask is subjected to a standard washing routine and dried under vacuum. The support from each flask is retrieved, thoroughly mixed together and redistributed in equal-size portions in the 16 flasks. The next step is the Mitsunobu alkylation with four different active methylene compounds (A1-A4) with five equivalents each of tributyl phosphine and TMAD in THE at room temperature. Each of the four reagents, A1-A4, are delivered to a row of four flasks. After 12hr of shaking at room temperature, excess reagents are drained and the support is subjected to washing and drying. The support from each flask is again retrieved, mixed and redistributed into the flasks. Finally, cyclization of the Mitsunobu alkylation products is conducted with hydrazine derivatives (H1-H4) in refluxing toluene. Each of the four reagents, H1-H4, are delivered to a row of four flasks. The antipyretic compounds are cleaved with 20% TFA in DCM and isolated by aspiration of the compounds from the insoluble support. Each of the 16 reaction flasks contains a mixture of 16 compounds. There are 64 total number of unique compounds in the array in this experiment.

## Claims

1. A solid phase Mitsunobu alkylation reaction comprising: 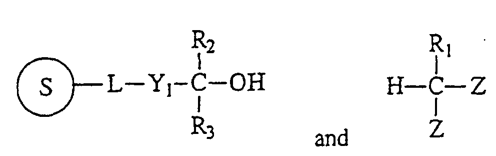 whereby product **I** results: 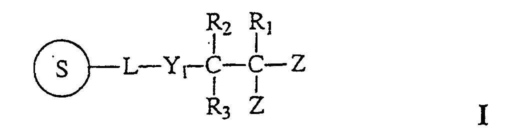 or, 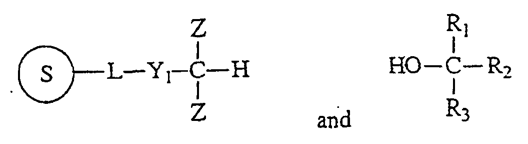 whereby product **II** results: 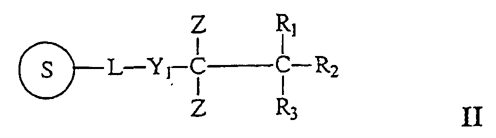 or, 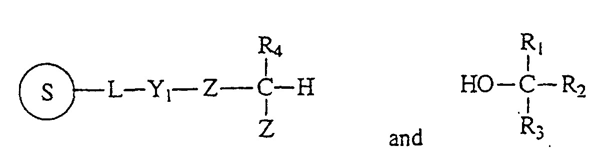 whereby product **III** results: 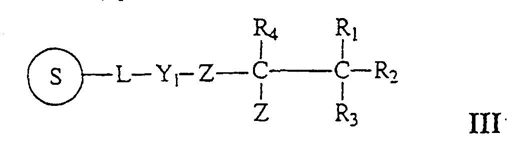 wherein;
S is a solid support,
L is a linker,
Y₁ is a substituent selected from the group consisting of substituted lower alkyl, substituted alkenyl, substituted alkynyl, and substituted aryl,
R₁, R₂, R₃, and R₄ are selected from the group consisting of H, C₁-C₂₀-alkyl, aryl, and halogen, and
Z is an electron-withdrawing group;
wherein the reaction is conducted in the presence of an azo-containing compound and a trivalent phosphorus compound.

2. The process of claim 1 wherein impurities and excess reagents are separated from the products **I, II, III** by filtration, aspiration, withdrawing of liquids, precipitation, or evaporation.

3. The process of claim 1 wherein Z is selected from the group consisting of carboxylic acid, ester, amide, alkylketone, arylketone, carbonyl, aldehyde, cyano, aldehyde, alkylsulfone, arylsulfone, sulfone, sulfoxide, sulfonamide, sulfonate, nitro, aryl, phosphonate, and phosphate.

4. The process of claim 1 wherein the azo-containing compounds are selected from the group consisting of tetramethylazodicarboxamide or diethylazodicarboxylate.

5. The process of claim 1 wherein R of the trivalent phosphorus reagents are selected from the group consisting of C₁-C₂₀-alkyl, phenyl, aryl, C₁-C₂₀-alkoxy, phenoxy, and aryloxy.

6. The process of claim 5 wherein trivalent phosphorus reagents are selected from the group consisting of tributylphosphine, triethylphosphine or triphenylphosphine.

7. The process of claim 1 wherein S is selected from the group consisting of polystyrene, controlled-pore-glass, polyacrylate, hydroxethylmethacrylate, polyamide, polyethylene, polyethyleneoxy, or copolymers and grafts of such.

8. The process of claim 1 wherein S is comprised of non-porous surfaces.

9. The process of claim 1 wherein L is selected from the group consisting of -NH, O, S, and CO₂.

10. The process of claim 1 wherein Y₁ is selected from the group consisting of

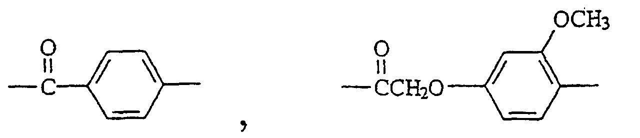

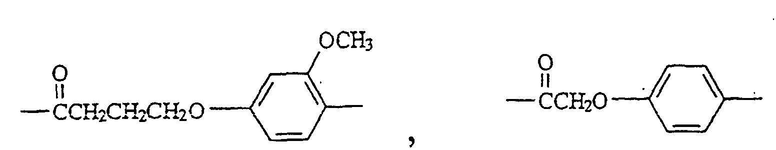

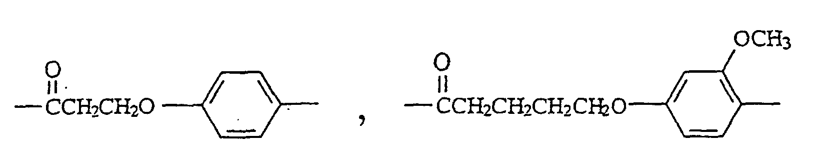

11. The process of claim 1 further comprising the step of cleaving a compound from a solid phase support comprising **I, II,** and **III** with cleaving reagents whereby compounds result comprising 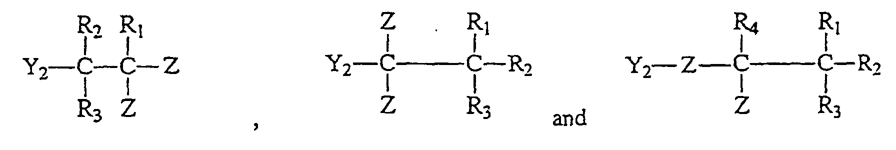 wherein;
Y₂ is selected from the group consisting of amino, hydroxy, carboxy, carboxylic ester, substituted C₁-C₂₀-alkyl and substituted aryl,
R₁, R₂, R_{3,} and R₄ are selected from the group consisting of H, C₁-C₂₀-alkyl, aryl, halogen,
Z is selected from the group consisting of electron-withdrawing groups.

12. The process of claim 1 further comprising the steps of:
a) distributing solid phase supports comprising **I, II,** and **III** in individually addressable locations on an array,
b) reacting compounds comprising: 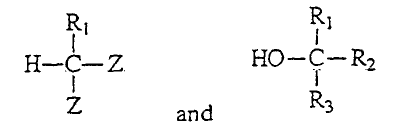 wherein; S, L, Y₁, R₁, R₂, R₃, R₄, and Z may be the same or different and the reactions are conducted substantially in parallel,
c) separating impurities and excess reagents from the product by filtration, aspiration, withdrawing liquids, precipitation, or evaporation.
d) cleaving the resulting products substantially in parallel, and
e) collecting, identifying, or analyzing said products in said locations.

13. The process of claim 1 further comprising the steps:
a) distributing solid phase supports comprising **I, II,** and **III** in individually addressable locations on an array,
b) reacting compounds comprising: 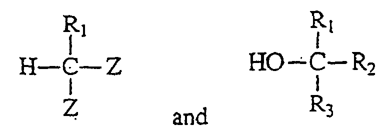 wherein; S, L, Y₁, R₁, R₂, R₃, R₄, and Z may be the same or different and the reactions are conducted substantially in parallel,
c) separating impurities and excess reagents from the product by filtration, aspiration, withdrawing liquids, precipitation, or evaporation.
d) combining and mixing the solid phase supports,
e) distributing a portion of the combined supports in said locations,
f) cleaving the resulting products substantially in parallel, and
g) collecting, identifying, or analyzing said products in said locations.

14. The process of claim 1 wherein said process is performed simultaneously on a plurality of solid phase supports having individually addressable locations on an array.

15. The process of claim 14 wherein after said contacting, at least one support-bound product is cleaved to release an adduct of the form 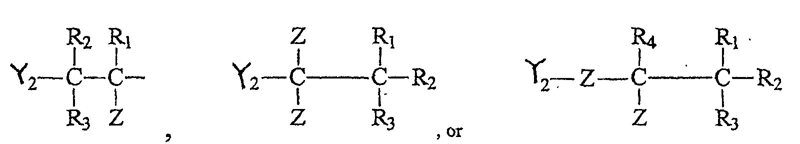 wherein Y₂ is selected from the group consisting of amino, hydroxy, carboxy, carboxylic ester, substituted or unsubstituted C₁-C₂₀-alkyl and substituted or unsubstatuted aryl.

16. The process of claim 1, wherein (a) said process is performed simultaneously on a plurality of solid phase supports having individually addressable locations on an array, (b) after said contacting, said solid phase supports are mixed together and then divided into a plurality of aliquots.

17. The process of claim 16 wherein subsequent to said dividing, at least one support-bound product is cleaved to release an adduct of the form 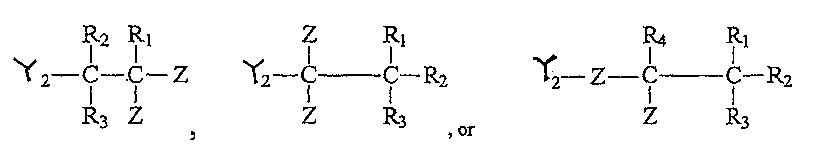 wherein Y₂ is selected from the group consisting of amino, hydroxy, carboxy, carboxylic ester, substituted or unsubstituted C₁-C₂₀-alkyl and substituted or unsubstituted aryl.

## Patentansprüche

1. Festphasen-Mitsunobu-Alkylierungsreaktion, umfassend: 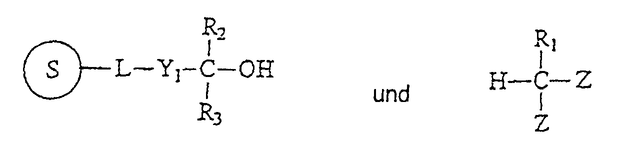 wobei sich Produkt I ergibt: 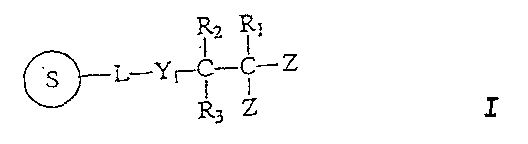 oder 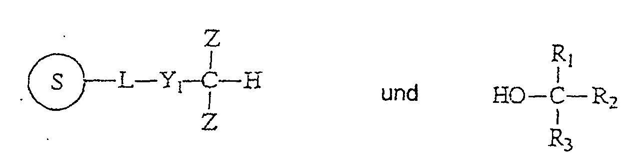 wobei sich Produkt II ergibt: 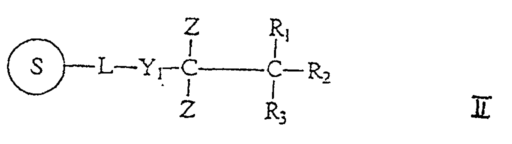 oder 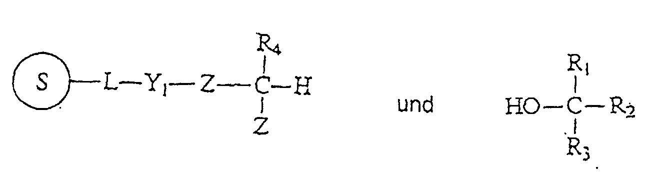 wobei sich Produkt III ergibt: 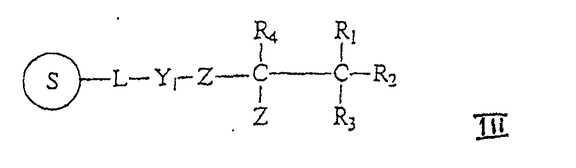 wobei
S eine Festphase ist,
L ein Linker ist,
Y₁ ein Substituent ist, ausgewählt aus der Gruppe bestehend aus einer substituierten niederen Alkyl-, substituierten Alkenyl-, substituierten Alkinyl- und substituierten Arylgruppe,
R₁, R₂, R₃ und R₄ ausgewählt werden aus der Gruppe bestehend aus H, C₁-C₂₀-Alkyl-, Arylgruppe und Halogenatom, und
Z eine elektronenziehende Gruppe ist;
wobei die Reaktion in Gegenwart einer azohaltigen Verbindungen und einer trivalenten Phosphorverbindung erfolgt.

2. Verfahren nach Anspruch 1, wobei Verunreinigungen und Überschussreagenzien von den Produkten I, II, III durch Filtration, Aspiration, Entfernung von Flüssigkeiten, Präzipitation oder Verdampfung getrennt werden.

3. Verfahren nach Anspruch 1, wobei Z ausgewählt wird aus der Gruppe bestehend aus Carbonsäure-, Ester-, Amid-, Alkyketon-, Arylketon-, Carbonyl-, Aldehyd-, Cyano-, Aldehyd-, Alkylsulfon-, Arylsulfon-, Sulfon-, Sulfoxid, Sulfonamid-, Sulfonat-, Nitro-, Aryl-, Phosphonatund Phosphatgruppe.

4. Verfahren nach Anspruch 1, wobei die azohaltigen Verbindungen ausgewählt werden aus der Gruppe bestehend aus Tetramethylazodicarboxamid oder Diethylazodicarboxylat.

5. Verfahren nach Anspruch 1, wobei R der trivalenten phosphorhaltigen Reagenzien ausgewählt wird aus der Gruppe bestehend aus C₁-C₂₀-Alkyl-, Phenyl-, Aryl-, C₁-C₂₀-Alkoxy-, Phenoxy- und Aryloxygruppe.

6. Verfahren nach Anspruch 5, wobei die trivalenten phosphorhaltigen Reagenzien ausgewählt werden aus der Gruppe bestehend aus Tributylphosphin, Triethylphosphin oder Triphenylphosphin.

7. Verfahren nach Anspruch 1, wobei S ausgewählt wird aus der Gruppe bestehend aus Polystyrol, kontrolliertem Porenglas, Polyacrylat, Hydroxyethylmethacrylat, Polyamid, Polyethylen, Polyethylenoxid oder Copolymeren und Pfropf-Copolymeren.

8. Verfahren nach Anspruch 1, wobei S aus nicht-porösen Oberflächen besteht.

9. Verfahren nach Anspruch 1, wobei L ausgewählt wird aus der Gruppe bestehend aus NH, O, S und CO₂.

10. Verfahren nach Anspruch 1, wobei Y₁ ausgewählt wird aus der Gruppe bestehend aus

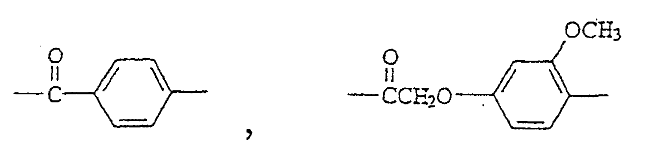

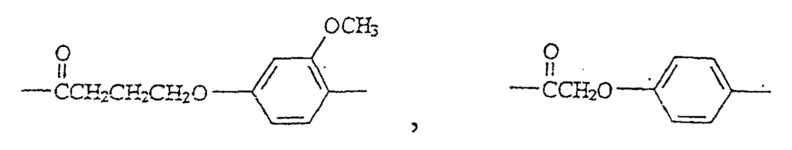

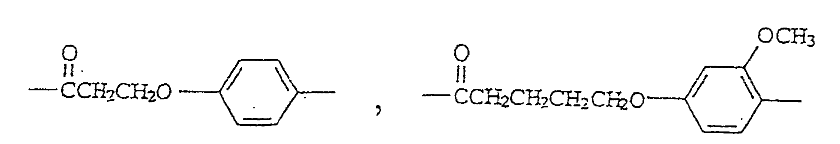

11. Verfahren nach Anspruch 1, das weiter den Schritt des Abspaltens einer Verbindung von einem Festphasenträger, umfassend I, II und III, mit Spaltungsreagenzien umfasst, wobei sich Verbindungen ergeben, umfassend 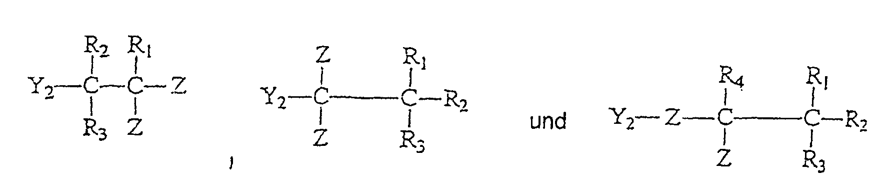 wobei
Y₂ ausgewählt wird aus der Gruppe bestehend aus Amino-, Hydroxy-, Carboxy-, Carbonsäureester-, substituierte C₁-C₂₀-Alkyl- und substituierte Arylgruppe,
R₁, R₂, R₃ und R₄ ausgewählt werden aus der Gruppe bestehend aus H, C₁-C₂₀-Alkyl-, Arylgruppe und Halogenatom,
Z ausgewählt wird aus der Gruppe bestehend aus elektronenziehenden Gruppen.

12. Verfahren nach Anspruch 1, umfassend weiter die Schritte:
a) Verteilen von Festphasenträgern umfassend I, II und III auf individuell adressierbare Positionen auf einer Anordnung,
b) Umsetzen der Verbindungen umfassend: 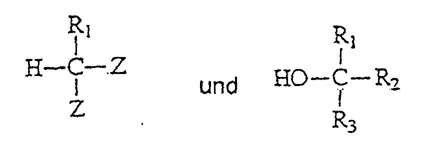 wobei S, L, Y₁, R₁, R₂, R₃, R₄ und Z gleich oder verschieden sein können und die Reaktionen im Wesentlichen parallel erfolgen,
c) Abtrennen von Verunreinigungen und Überschussreagenzien von dem Produkt durch Filtration, Aspiration, Abziehen von Flüssigkeiten, Präzipitation oder Verdampfung,
d) Abspalten der erhaltenen Produkte im Wesentlichen gleichzeitig und
e) Sammeln, Identifizieren oder Analysieren der Produkte auf den Positionen.

13. Verfahren nach Anspruch 1, umfassend weiterhin die Schritte:
a) Verteilen von Festphasenträgern umfassend I, II und III auf individuell adressierbare Positionen auf einer Anordnung,
b) Umsetzen der Verbindungen umfassend: 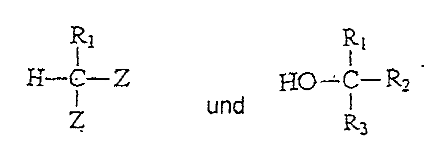 wobei S, L, Y₁, R₁, R₂, R₃, R₄ und Z gleich oder verschieden sein können und die Reaktionen im Wesentlichen gleichzeitig erfolgen,
c) Abtrennen von Verunreinigungen und Überschussreagenzien von dem Produkt durch Filtration, Aspiration, Abziehen von Flüssigkeiten, Präzipitation oder Verdampfung,
d) Vereinigen und Mischen der Festphasenträger,
e) Verteilen eines Teils der vereinigten Träger auf die Positionen,
f) Spalten der erhaltenen Produkte im Wesentlichen gleichzeitig und
g) Sammeln, Identifizieren oder Analysieren der Produkte auf den Positionen.

14. Verfahren nach Anspruch 1, wobei das Verfahren simultan an einer Vielzahl von Festphasenträgern mit individuell adressierbaren Positionen auf einer Anordnung erfolgt.

15. Verfahren nach Anspruch 14, wobei nach dem in-Kontakt-Bringen mindestens ein trägergebundenes Produkt gespalten wird, um ein Addukt der Form 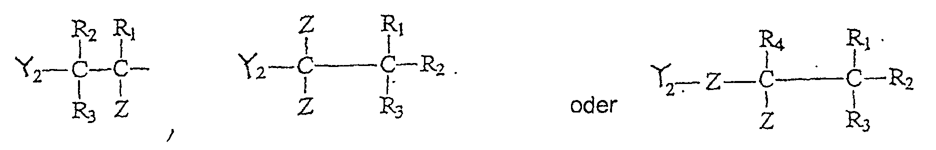 freizusetzen, wobei Y₂ ausgewählt wird aus der Gruppe bestehend aus Amino-, Hydroxy-, Carboxy-, Carbonsäureester-, substituierter oder unsubstituierter C₁-C₂₀-Alkyl- und substituierter oder unsubstituierter Arylgruppe.

16. Verfahren nach Anspruch 1, wobei (a) das Verfahren simultan an einer Vielzahl von Festphasenträgern mit individuell adressierbaren Positionen auf einer Anordnung erfolgt, (b) nach dem In-Kontakt-Bringen die Festphasenträger zusammengemischt und dann in eine Vielzahl von Aliquots aufgeteilt werden.

17. Verfahren nach Anspruch 16, wobei sofort nach der Teilung mindestens ein trägergebundenes Produkt gespalten wird, um ein Addukt der Form 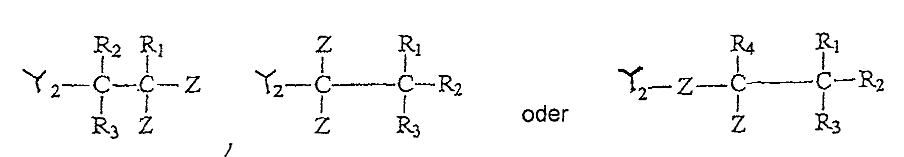 freizusetzen, wobei Y₂ ausgewählt wird aus der Gruppe bestehend aus Amino-, Hydroxy-, Carboxy-, Carbonsäureester-, substituierter oder unsubstituierter C₁-C₂₀-Alkyl- und substituierter oder unsubstituierter Arylgruppe.

## Revendications

1. Réaction d'alkylation de Mitsunobu en phase solide comprenant: 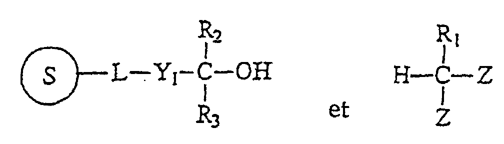 conduisant au produit I: 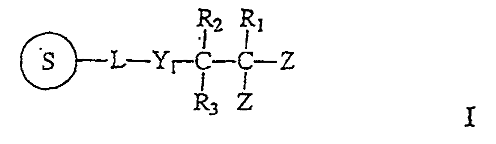 ou 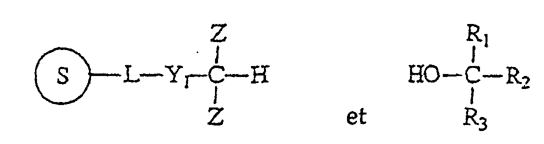 conduisant au produit II: 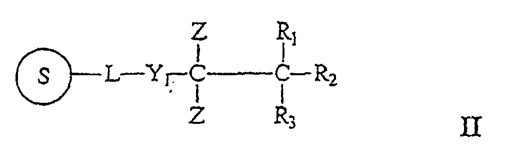 ou 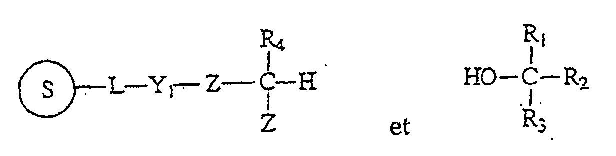 conduisant au produit III: 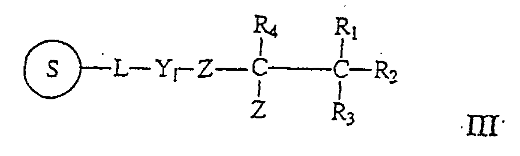 où:
S est un support solide,
L est un lieur,
Y₁ est un substituant choisi dans le groupe consistant en alkyle inférieur substitué, alcényle substitué, alcynyle substitué et aryle substitué,
R₁, R₂, R₃ et R₄ sont choisis dans le groupe consistant en H, alkyle en C₁-C₂₀, aryle et halogéno, et
Z est un groupe d'enlèvement d'électrons;
tandis que la réaction est conduite en présence d'un composé contenant des groupes azoïques et d'un composé de phosphore trivalent.

2. Procédé selon la revendication 1, dans lequel les impuretés et les réactifs en excès sont séparés des produits I, II, III par filtration, aspiration, extraction de liquides, précipitation ou évaporation.

3. Procédé selon la revendication 1, dans lequel Z est choisi dans le groupe consistant en acide carboxylique, ester, amide, alkylcétone, arylcétone, carbonyle, aldéhyde, cyano, aldéhyde, alkylsulfone, arylsulfone, sulfone, sulfoxyde, sulfonamide, sulfonate, nitro, aryle, phosphonate et phosphate.

4. Procédé selon la revendication 1, dans lequel les composés contenant des groupes azoïques sont choisis dans le groupe consistant en tétraméthylazodicarboxamide ou diéthylazodicarboxylate.

5. Procédé selon la revendication 1, dans lequel les R des réactifs à phosphore trivalent sont choisis dans le groupe consistant en alkyle en C₁-C₂₀, phényle, aryle, alcoxy en C₁-C₂₀, phénoxy et aryloxy.

6. Procédé selon la revendication 5, dans lequel les réactifs à phosphore trivalent sont choisis dans le groupe consistant en tributylphosphine, triéthylphosphine ou triphénylphosphine.

7. Procédé selon la revendication 1, dans lequel S est choisi dans le groupe consistant en polystyrène, verre à pores maîtrisés, polyacrylate, hydroxyéthylméthacrylate, polyamide, polyéthylène, polyéthylèneoxy ou des copolymères et des produits de greffage de ceux-ci.

8. Procédé selon la revendication 1, dans lequel S est composé de surfaces non-poreuses.

9. Procédé selon la revendication 1, dans lequel L est choisi dans le groupe consistant en NH, O, S et CO₂.

10. Procédé selon la revendication 1, dans lequel Y₁ est choisi dans le groupe consistant en

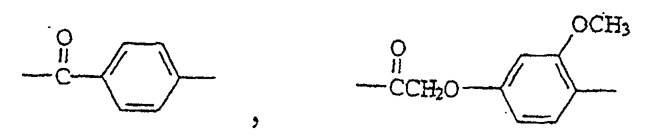

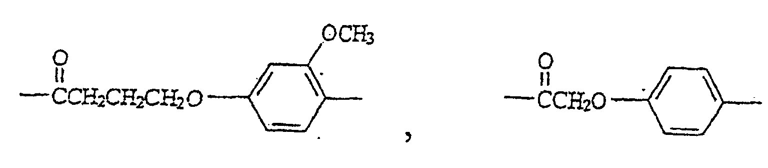

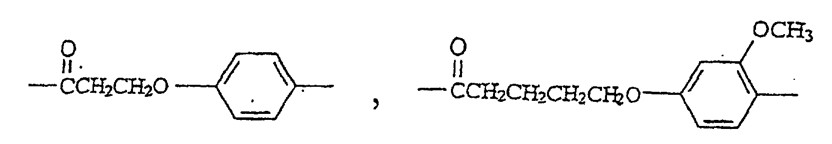

11. Procédé selon la revendication 1, comprenant en outre l'étape de clivage d'un composé d'un support en phase solide comprenant I, II et III avec des réactifs de clivage, avec pour résultat des composés comprenant 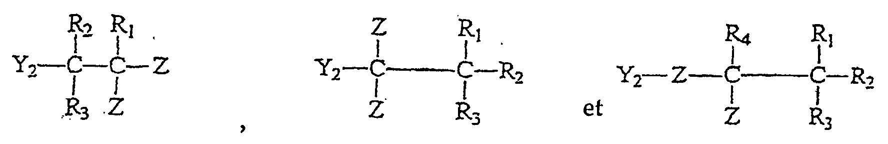 où:
Y₂ est choisi dans le groupe consistant en amino, hydroxy, carboxy, ester carboxylique, alkyle en C₁-C₂₀ substitué et aryle substitué,
R₁, R₂, R₃ et R₄ sont choisis dans le groupe consistant en H, alkyle en C₁-C₂₀, aryle, halogéno,
Z est choisi dans le groupe consistant en des groupes d'enlèvement d'électrons.

12. Procédé selon la revendication 1, comprenant en outre les étapes de:
a) répartition de supports en phase solide comprenant I, II et III dans des emplacements adressables individuellement sur une matrice,
b) réaction de composés comprenant: 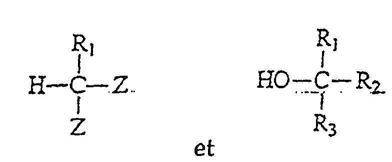 où: S, L, Y₁, R₁, R₂, R₃, R₄ et Z peuvent être identiques ou différents et les réactions sont conduites substantiellement en parallèle,
c) séparation d'impuretés et de réactifs en excès du produit par filtration, aspiration, extraction de liquides, précipitation ou évaporation,
d) clivage des produits résultants, substantiellement en parallèle, et
e) recueil, identification ou analyse des dits produits dans lesdits emplacements.

13. Procédé selon la revendication 1, comprenant en outre les étapes de:
a) répartition de supports en phase solide comprenant I, II et III dans des emplacements adressables individuellement sur une matrice,
b) réaction de composés comprenant: 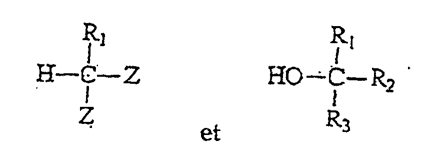 où: S, L, Y₁, R₁, R₂, R₃, R₄ et Z peuvent être identiques ou différents et les réactions sont conduites substantiellement en parallèle,
c) séparation d'impuretés et de réactifs en excès du produit par filtration, aspiration, extraction de liquides, précipitation ou évaporation,
d) combinaison et mélange des supports en phase solide,
e) répartition d'une portion des supports combinés dans lesdits emplacements,
f) clivage des produits résultants, substantiellement en parallèle, et
g) recueil, identification ou analyse des dits produits dans lesdits emplacements.

14. Procédé selon la revendication 1, dans lequel ledit procédé est mis en oeuvre simultanément sur une pluralité de supports en phase solide ayant des emplacements adressables individuellement sur une matrice.

15. Procédé selon la revendication 14, dans lequel après ladite mise en contact, au moins un produit lié à un support est clivé pour libérer un produit d'addition de la forme 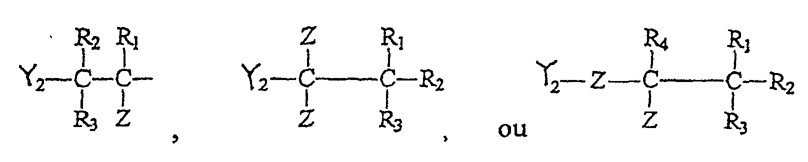 où Y₂ est choisi dans le groupe consistant en amino, hydroxy, carboxy, ester carboxylique, alkyle en C₁-C₂₀ substitué ou non-substitué et aryle substitué ou non-substitué.

16. Procédé selon la revendication 1, dans lequel (a) ledit procédé est mis en oeuvre simultanément sur une pluralité de supports en phase solide ayant des emplacements adressables individuellement sur une matrice, (b) après ladite mise en contact, lesdits supports en phase solide sont mélangés ensemble et ensuite divisés en une pluralité de fractions aliquotes.

17. Procédé selon la revendication 16, dans lequel à la suite de ladite division, au moins un produit lié à un support est clivé pour libérer un produit d'addition de la forme 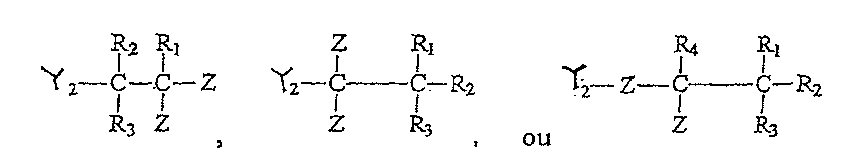 où Y₂ est choisi dans le groupe consistant en amino, hydroxy, carboxy, ester carboxylique, alkyle en C₁-C₂₀ substitué ou non-substitué et aryle substitué ou non-substitué.
